(19) **Europäisches Patentamt / European Patent Office / Office européen des brevets**

(11) **EP 2 171 472 B1**

(12) # EUROPEAN PATENT SPECIFICATION

(45) Date of publication and mention of the grant of the patent:
**14.12.2011 Bulletin 2011/50**

(51) Int Cl.:
***G01N 33/66*** (2006.01)

(21) Application number: **08771919.1**

(22) Date of filing: **25.06.2008**

(86) International application number:
**PCT/US2008/068176**

(87) International publication number:
**WO 2009/006155 (08.01.2009 Gazette 2009/02)**

(54) **IN VITRO METHOD FOR THE DETERMINATION OF GLYCEMIC INDEX OF FOOD PRODUCTS**

IN-VITRO-VERFAHREN ZUR BESTIMMUNG DES GLYKÄMISCHEN INDEX VON LEBENSMITTELPRODUKTEN

PROCÉDÉ IN VITRO DE DÉTERMINATION DE L'INDICE GLYCÉMIQUE DE PRODUITS ALIMENTAIRES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.06.2007 US 770361**
**23.06.2008 US 144056**

(43) Date of publication of application:
**07.04.2010 Bulletin 2010/14**

(73) Proprietor: **Kraft Foods Global Brands LLC Northfield, IL 60093 (US)**

(72) Inventors:
- **MAGALETTA, Robert, L.**
  **Branchburg, New Jersey 08876 (US)**
- **DICATALDO, Suzanne, N.**
  **Florham Park, New Jersey 07932 (US)**

(74) Representative: **Setna, Rohan P.**
**Boult Wade Tennant**
**Verulam Gardens**
**70 Gray's Inn Road**
**London WC1X 8BT (GB)**

(56) References cited:
- **ENGLYST KLAUS N ET AL: "Rapidly available glucose in foods: An in vitro measurement that reflects the glycemic response" AMERICAN JOURNAL OF CLINICAL NUTRITION, BETHESDA,MD, US, vol. 69, no. 3, 1 March 1999 (1999-03-01), pages 448-454, XP002353205 ISSN: 0002-9165 cited in the application**
- **FLINT ANNE ET AL: "The use of glycaemic index tables to predict glycaemic index of composite breakfast meals" BRITISH JOURNAL OF NUTRITION, vol. 91, no. 6, June 2004 (2004-06), pages 979-989, XP008096523 ISSN: 0007-1145**
- **TROUT DAVID ET AL: "Prediction of glycemic index among high-sugar, low-starch foods" INTERNATIONAL JOURNAL OF FOOD SCIENCES AND NUTRITION, vol. 50, no. 2, March 1999 (1999-03), pages 135-144, XP008096747 ISSN: 0963-7486**

**Description**

**Field of the Invention**

The present invention relates to an *in vitro* method for determining the glycemic index (GI) values for various food products. The present invention provides an accurate and inexpensive *in vitro* method for determining the glycemic index of a wide variety of both food ingredients and finished food products.

**Background of the Invention**

Interest in the glycemic index of food products has significantly increased in recent years. The glycemic index is an indicator of the relative glycemic response to dietary carbohydrates in a given food product upon human digestion and allows foods to be ranked based on the rate of release and absorption of carbohydrates. In effect, the glycemic index allows identification of so-called "good carbs" (i.e., carbohydrates with relatively low glycemic indexes) and so-called "bad carbs" (i.e., carbohydrates with relatively high glycemic indexes). Carbohydrate-conscious consumers and health care providers can use the glycemic index or related values to assist in food selections.

The glycemic index ranks carbohydrates on a scale of 0 to 100 based on changes in blood sugar levels after eating. Foods with a high GI are thought to be rapidly digested and absorbed, thereby leading to marked fluctuations in blood sugar levels. Low glycemic index values are though to produce more gradual rises, and thus flattened fluctuations, in blood sugar and insulin levels due to their slower digestion and absorption by the body. Generally, low glycemic index foods are defined as having a glycemic index of 55 or less, medium glycemic index foods as having a glycemic index of 56 to 69, and high glycemic index foods as having a glycemic index of 70 or higher.

The consumption of high-glycemic index foods generally appears to result in higher and more rapid increases in blood glucose levels than the consumption of low-glycemic index foods. Rapid increases in blood glucose signal the pancreas to increase insulin secretion. High insulin levels induced by consumption of high-glycemic index foods may cause a sharp decrease in blood glucose levels (hypoglycemia) whereas consumption of low-glycemic index foods are generally thought to result in lower and more sustained increases in blood glucose and lower insulin demands. Low glycemic diets have been reported to improve both glucose and lipid levels in people with diabetes (type 1 and type 2). Low glycemic diets have also been reported to result in benefits in weight control because they help control appetite and delay hunger. Low GI diets may also reduce insulin levels and insulin resistance. Recent studies from Harvard School of Public Health report that the risks of diseases such as type 2 diabetes and coronary heart disease are strongly influenced by the glycemic index of the overall diet. The World Health Organization (WHO) and Food and Agriculture Organization of the United Nations have recommended that people in industrialized countries base their diets on low glycemic index foods in order to prevent the most common diseases of affluence, such as coronary heart disease, diabetes, and obesity. Thus, it is often recommended that consumers modify their overall diets such that the relative amount of low glycemic index foods is increased at the expense of high glycemic index foods. Glycemic index values can be used to assist consumers and health care providers in selecting foods and possibly reducing the risk of certain diseases.

The glycemic index of a given food product is usually determined *in vivo* by monitoring the blood glucose level of a group of human subjects (usually about 6 or more individuals) who have ingested the food product; the blood glucose response for the food product is compared to that stimulated by ingestion of a control substance of known glycemic index over a fixed period of time and the glycemic index is calculated. The *in vivo* glycemic index determination method is generally considered the "gold standard" in this area. In the currently accepted test protocol, measured portions of a test food containing 10 to 50 grams of carbohydrate are fed to 6 or more healthy people after an overnight fast. Blood samples, usually from finger-pricks, are taken before the food consumption (time zero) and at 15-30 minute intervals in the two hours immediately after the food consumption and analyzed for blood glucose levels. The resulting data (typically about 7 data points) are used to prepare a blood sugar response curve (i.e., blood glucose level plotted against time) for the two hour period after consumption of the test food. The area under the blood sugar curve is related to the total rise in blood glucose levels after eating the test food. A similar test is carried out, again with overnight fasting, with the same individuals consuming an equal-carbohydrate portion of glucose sugar (the reference food having, by definition, a glycemic index of 100) or white bread having a defined GI value; the two-hour blood glucose response curves are determined and the area under the curves is measured in the same manner as done for the test food. The glycemic index of the test food is calculated by dividing the area under the curve for the test food by the area under the curve for the reference food and multiplying by 100. The use of a standard food is important for reducing the confounding influence of differences in the physical and/or other characteristics of the subjects as well as to match the physical form of the test samples (i.e., standard aqueous glucose solution for beverage determinations and standard white bread for solid food determinations). Since only about 7 data points (over a two hour period) are used to generate the blood sugar curve and the curve is assumed to pass smoothly through these limited data points, errors could arise if the glucose generation is significantly increased or decreased between the actual data points taken.

The average of the glycemic indexes from all test subjects is taken as the glycemic index of the food. Of course, the accuracy of the determination depends, at least in part, on compliance of the test subjects regarding the test protocols and the validity of the assumption that the physical and/or other characteristics of the individual subjects remains essentially constant for both the test and reference food determinations.

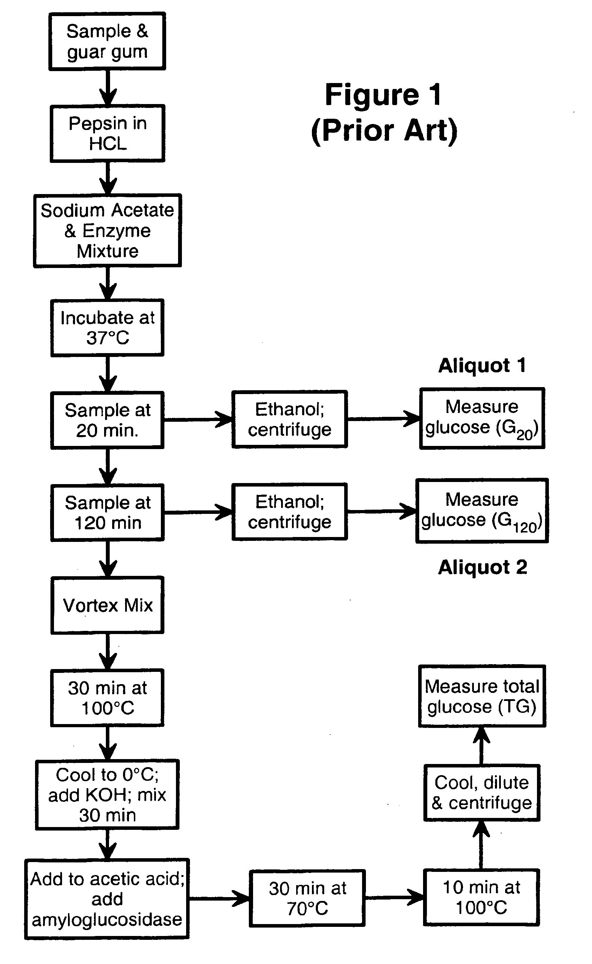

Since such *in vivo* glycemic index determination methods generally require human subjects as well as being costly and time consuming, there has been considerable interest in developing *in vitro* test protocols. One *in vitro* method, based on studies by K.N. Englyst and co-workers and illustrated in Figure 1, involved the measurement of glucose released from a test food during timed incubation at 37°C with a mixture of digestive enzymes using a colorimetric endpoint to determine the glucose level. Englyst et al., Brit. J. Nutr., 75, 327-337 (1996). This *in vitro* method has more recently been modified to include a HPLC endpoint to determine the amount of glucose released. Englyst et al., Am. J. Clin. Nutr., 69, 448-454 (1999) (hereinafter referred to as the "Englyst method" or the *"in vitro* Englyst method").

The Englyst method involves mincing (or otherwise crushing or breaking up) a known amount of a test food (generally to contain about 0.5 g carbohydrate). The minced samples are incubated at 37°C for 30 minutes with mixing in 10 ml 0.05 M HCl containing pepsin (5 g/l; to effect hydrolysis of protein) and guar gum (5 g/l; to help maintain food particles in suspension throughout the analysis). After this initial incubation, the samples are buffered to pH 5.2 using 0.5 M sodium acetate. An enzyme mixture (containing specific amounts of pancreatin, amyloglucosidase, and invertase) is then added to the buffered sample and the sample placed in a shaking water bath at 37°C (time = 0). Small glass balls are included in the samples to mechanically disrupt the physical structure of the samples during the main incubation; the added guar gum helps keep the sample in suspension by stabilizing the viscosity of the samples. At exactly 20 minutes into the main incubation, an aliquot of the sample is removed and added to absolute ethanol with mixing to stop the hydrolysis; this sample is used to determine $G_{20}$ (i.e., the glucose released after 20 minutes; also referred to as "rapidly available glucose" or RAG) using HPLC. The remainder of the sample is maintained in the 37°C bath for an additional 100 minutes at which time a second aliquot of the sample is removed and added to absolute ethanol with mixing to stop the hydrolysis; this sample is used to determine $G_{120}$ (i.e., the glucose released after 120 minutes) using HPLC. The remainder of the sample is then treated as illustrated in Figure 1 by the addition of additional enzymes and then exposure to temperatures up to 100°C to force complete hydrolysis and, thus, determine the total glucose in the sample.

Comparing foods for which the glycemic index has been measured using the *in vivo* test method, the $G_{20}$ or rapidly available glucose values determined using the Englyst method can be correlated with the *in vivo* glycemic index values of known foods. Using such correlation coefficients, the Englyst method can be used to estimate glycemic index values for test foods.

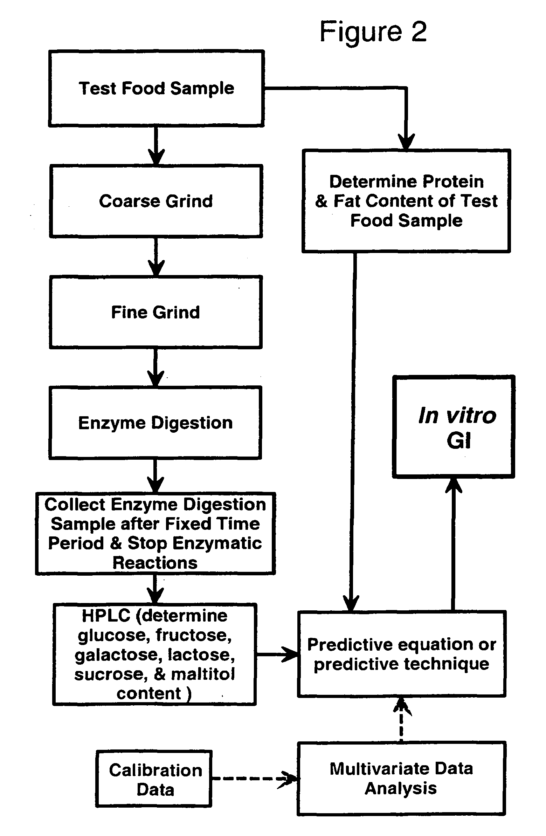

The *in vitro* Englyst method has, however, met with considerable criticism, especially from proponents of the *in vivo* glycemic index method. For example, Garsetti et al., J. Am. Coll. Nutr., 24, 441-447 (2005), used the Englyst method to estimate glycemic index values (i.e., rapidly available glucose values) and then compared them with *in vivo* determined values for food products (i.e., cookies) having glycemic indexes in the range of about 35 to 60. As shown in Fig. 2 of Garsetti et al., a scatter plot of *in vivo* determined glycemic index values versus rapidly available glucose values (i.e., as determined by the Englyst method) yielded a scatter plot with an $R^2$ value of 0.25, indicating that the *in vitro* method had little predictive value.

Brand-Miller et al., Eur. J. Clin. Nutr., 58, 700-701 (2004), determined the *in vivo* glycemic index values for commercially available foods (one breakfast cereal and two snack bars) reportedly having low glycemic index values (i.e., $\leq 55$) as determined by the Englyst *in vitro* test method. According to Brand-Miller et al., the three food products had mean *in vivo* glycemic index values ($\pm$ sem) of $75 \pm 3$, $68 \pm 5$, and $65 \pm 5$, respectively, and thus could not be classified as low glycemic index food products. These authors concluded that assumptions that the "laborious task of *in* vivo testing is no longer necessary for measuring the GI values for food" are simply incorrect and "strongly advise against the use of any *in vitro* method for producing GI values for food labeling purposes." Finally, the authors "urge food manufactures to undertake GI testing only with experienced laboratories using the standardized *in vivo* method." "The use of glycaemic index tables to predict glycaemic index of composite breakfast materials" by Flint Anne et al., in the British Journal of Nutrition, Volume 91, Number 6, June 2004, pages 979-989, investigates the predictability of measured GI in composite breakfast meals when calculated from table values. "Prediction of glycaemic index among high-sugar, low-starch foods" by Trout David et al., in the International Journal of Food Sciences and Nutrition, Volume 50, Number 2, March 1999, pages 135-144, discloses the use of a weighted mean of the glycemic index values of the constituent sugars of non-starchy food to give a rough estimate of the GI of that food. "Rapidly available glucose in foods; an in vitro measurement that reflects the glycemic response "by Englyst Claus N et al in American Journal of Clinical Nutrition, Bethsda, ND, US, Volume 69, No. 3, 1 March 1999, pages 448-454 hypothesises that rapidly available glucose is an important food-related determinant of glycemic response.

Clearly there remains a need in the art for an accurate, precise, and reproducible *in vitro* method for determining the glycemic index values of food products. Moreover, there remains a need for such a method which can be applied to a large variety of food products, including solid and liquid food products. The present invention provides such *in vitro* testing methods.

## Summary of the Invention

The present invention provides an *in vitro* analytical method which allows the improved prediction of the glycemic index of a food product from the levels of protein, fat, and sugars/sugar alcohols generated using a simulated digestion of the food product with enzymes. Current glycemic index determination methods generally require human subjects and are both costly and time consuming. The inventive method is capable of determining the glycemic index of up to about 15 food samples in one day by one analyst at significantly reduced costs relative to the traditional method using human subjects. Glycemic index values determined using the present inventive method show very high correlation with results obtained using the traditional test using human subjects. Moreover, this present inventive method can be used with a wide variety of food products, including solid food products as well as semi-solid food products (e.g., yogurt and the like) and liquid food products (e.g., beverages and the like).

The present invention provides an *in vitro* method for determining glycemic index for a test food product, said method comprising

(1) determining protein content and fat content of the test food product;
(2) providing the test food product in an essentially homogeneous and finely divided state to provide a test food sample;
(3) digesting an amount of the test food sample in the essentially homogeneous and finely divided state sufficient to provide a standardized amount of available carbohydrate with a mixture of digestive enzymes for a fixed period of time to provide a digested sample;
(4) treating the digested sample in its entirety immediately upon the fixed period of time to stop enzymatic reactions;
(5) determining amounts of glucose and at least two sugars or sugar alcohols selected from the group of fructose, galactose, lactose, sucrose, and maltitol in the digestive sample from step (4); and
(6) calculating the glycemic index of the test food product from data obtained in steps (1) and (5) for the test food product using a predictive equation or a predictive technique derived from multivariate analysis of calibration data for calibration samples wherein the calibration data is of same type and obtained in same manner as the data for the test food product determined in steps (1) and (5) and wherein the calibration samples have known *in vivo* glycemic index values.

The present invention also provides an *in vitro* method for determining glycemic index for a test food product, said method comprising

(1) determining protein content and fat content of the test food product;
(2) providing the test food product in an essentially homogeneous and finely divided state to provide a test food sample wherein the test food product is ground at essentially liquid nitrogen temperatures;
(3) digesting an amount of the test food sample in the essentially homogeneous and finely divided state sufficient to provide a standardized amount of available carbohydrate with a mixture of digestive enzymes for a fixed period of time to provide a digested sample;
(4) treating the digested sample in its entirety immediately upon the fixed period of time to stop enzymatic reactions;
(5) determining amounts of glucose and at least two sugars or sugar alcohols selected from the group of fructose, galactose, lactose, sucrose, and maltitol in the digestive sample from step (4); and
(6) calculating the glycemic index of the test food product from data obtained in steps (1) and (5) for the test food product using a predictive equation or a predictive technique derived from multivariate analysis of calibration data for calibration samples wherein the calibration data is of same type and obtained in same manner as the data for the test food product determined in steps (1) and (5) and wherein the calibration samples have known *in vivo* glycemic index values.

The present *in vitro* method can be used to determine glycemic index values of solid, semi-solid, and liquid food products with significantly improved accuracy and precision as compared to currently available *in vitro* methods. Indeed, the accuracy and precision obtainable by the current inventive method is comparable to commercially available *in vivo* methods but with significant savings in both time and costs.

For purposes of this invention, "essentially homogeneous and finely divided state" is intended to mean a degree of grinding equivalent to grinding the food item at temperatures sufficiently low such that the food item is a brittle solid. Generally, temperatures lower than about -40°C, temperatures preferably below about -78°C, and more preferably temperatures at about temperatures obtainable using liquid nitrogen (-196°C) are sufficient for grinding most food items which are solid or semi-solid at ambient temperatures. It is also generally preferred that ambient temperature solid materials are broken up or coarsely ground before such low temperature grinding. In one preferred embodiment, and especially for solid or semi-solid materials at ambient temperatures, grinding is accomplished using a liquid nitrogen-cooled grinding mill at or close to liquid nitrogen temperature. In many cases, liquid food products will not require any grinding since they are essentially homogeneous and the components are soluble in the carrier; if desired, however, such liquid food products may be also be ground at lower temperatures. Semi-solid materials may or may not require such low temperature grinding to obtain an essentially homogeneous and finely divided state. Thus, for example, a plain yogurt need not be further ground whereas a yogurt with fruit or other solid ingredients therein preferably is ground at low temperatures to obtain an essentially homogeneous and finely divided state. Further guidance on the required "homogeneous and finely divided state" can be found in the examples included with this specification.

Preferably the amounts of glucose and at least two sugars or sugar alcohols selected from the group of fructose, galactose, lactose, sucrose, and maltitol in the digestive sample from step (4) are determined using high performance liquid chromatography (HPLC) or equivalent quantitative methods. Preferably high performance ion chromatography (HPIC) is used to determine the amounts of sugars released during the treatment with enzymes. More preferably, the amounts of glucose, fructose, galactose, lactose, sucrose, and maltitol in the digestive sample from step (4) are determined using high performance ion chromatography (HPIC).

**Brief Description of the Figures**

Figure 1 provides a flow chart describing an *in vitro* prior art method (i.e., the Englyst method) for estimating glycemic index and related values.

Figure 2 provides a flow chart illustrating the *in vitro* method of the present invention.

Figure 3 provides typical chromatograms of a mixed standard (Panel A) and a typical unknown sample (Panel B) obtained in the *in vitro* method described in Example 1.

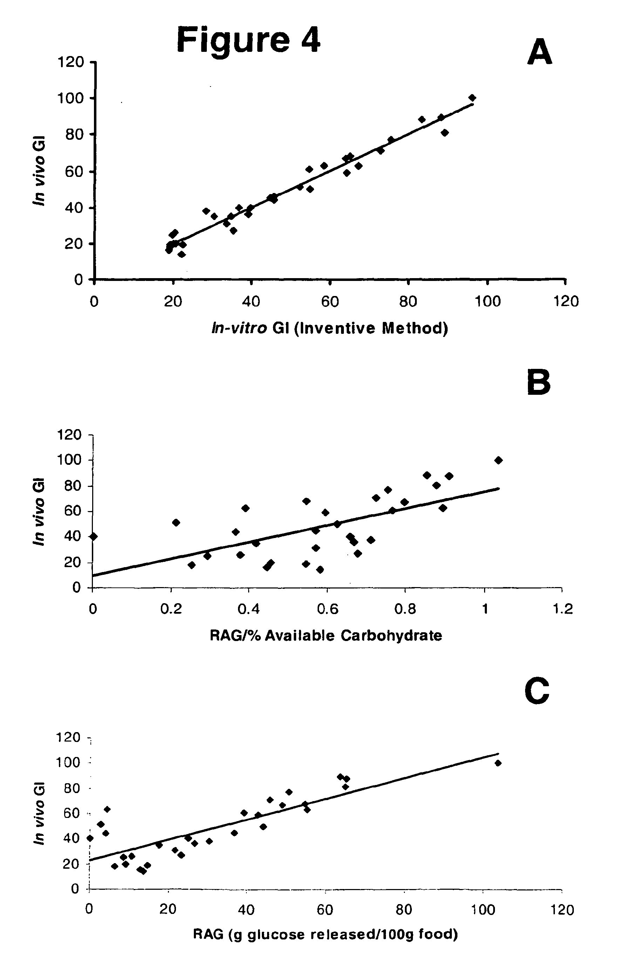

Figure 4A provides a comparison of the known *in vivo* glycemic index values and the *in vitro* glycemic index values determined using the inventive method with neural net curve fitting procedures based on the data in Example 2; the inventive method yielded a value of 0.97 for $R^2$. Figures 4B and 4C provide comparisons of the known *in vivo* glycemic index values and rapidly available glucose (RAG expressed as grams glucose released in 20 minutes/100 g food) as obtained using the calculation techniques used in the Englyst method (i.e., only based on the glucose release after 20 minutes digestion) with the data from Example 2. Figure 4B plots *in vivo* GI versus RAG/(% available carbohydrate); Figure 4C plots *in vivo* GI versus RAG. Using the Englyst calculation techniques on data set of Example 2 yielded $R^2$ values of 0.41 and 0.68 for the scatter plots shown in Figures 4B and 4C, respectively.

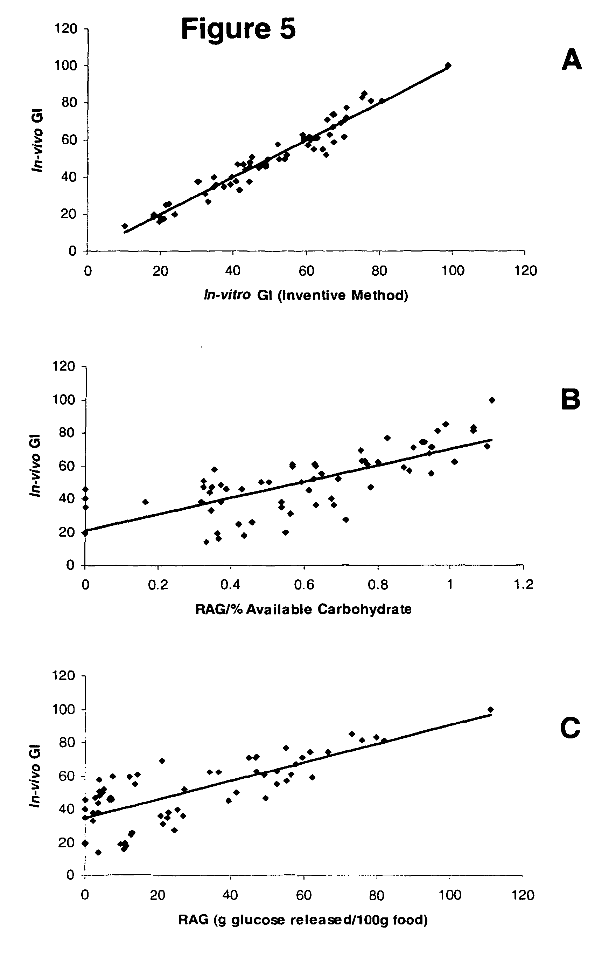

Figure 5A provides a comparison of the known *in vivo* glycemic index values and the *in vitro* glycemic index values determined using the inventive method with multiple linear regression curve fitting procedures based on the data in Example 3; the inventive method yielded a value of 0.94 for $R^2$. Figures 5B and 5C provide comparisons of the known *in vivo* glycemic index values and rapidly available glucose (RAG expressed as grams glucose released in 20 minutes/100 g food) as obtained using the calculation techniques used in the Englyst method (i.e., only based on the glucose release after 20 minutes digestion) with the data from Example 3. Figure 5B plots *in vivo* GI versus RAG/(% available carbohydrate); Figure 5C plots *in vivo* GI versus RAG. Using the Englyst calculation techniques on data set of Example 3 yielded $R^2$ values of 0.56 and 0.58, respectively, for the scatter plots shown in Figures 5B and 5C, respectively.

Figure 6 provides a comparison of the known *in vivo* glycemic index values and the *in vitro* glycemic index values determined using the inventive method with neural net curve fitting procedures based on the data in Example 4; the inventive method yielded a value of 0.96 for $R^2$.

**Detailed Description of the Invention**

The present invention provides an *in vitro* analytical method which allows the improved prediction of the glycemic index of a food product from the levels of protein, fat, and sugars/sugar alcohols liberated using a simulated digestion of the food product with enzymes. Current glycemic index determination methods generally require human subjects and are both costly and time consuming. The inventive method is capable of determining the glycemic index of up to about 15 food samples in one day by one analyst at significantly reduced costs relative to the traditional method using human subjects. Moreover, the administrative burden of screening human subjects, obtaining informed consent, and the like is avoided in the present invention. Glycemic index values determined using the present inventive method show very high correlation with results obtained using the traditional test with human subjects. Indeed, the *in vitro* method may yield results that are more precise than the results from the clinical *in vivo* method. Since the number of calibration samples used for the *in vitro* method is greater (and can continue to increase as additional calibration samples (i.e., samples for which the with known *in vivo* GI values) are incorporated therein) than that used for a single determination using the *in vivo* method, the *in vivo* data used to calibrate the *in vitro* method, from which the *in vitro* GI results are derived, are based on the blood glucose level results of a far larger number of human subjects than that used in any single in *vivo* test. Moreover, this present inventive method can be used with a wide variety of food products, including solid food products as well as semi-solid food products (i.e., yogurt and the like) and liquid food products (i.e., beverages and the like).

The present invention provides an *in vitro* method for determining glycemic index for a test food product, said method comprising

(1) determining protein content and fat content of the test food product;
(2) providing the test food product in an essentially homogeneous and finely divided state to provide a test food sample;
(3) digesting an amount of the test food sample in the essentially homogeneous and finely divided state sufficient to provide a standardized amount of available carbohydrate with a mixture of digestive enzymes for a fixed period of time to provide a digested sample;
(4) treating the digested sample in its entirety immediately upon the fixed period of time to stop enzymatic reactions;
(5) determining amounts of glucose and at least two sugars or sugar alcohols selected from the group of fructose, galactose, lactose, sucrose, and maltitol in the digestive sample from step (4); and
(6) calculating the glycemic index of the test food product from data obtained in steps (1) and (5) for the test food product using a predictive equation or a predictive technique derived from multivariate analysis of calibration data for calibration samples wherein the calibration data is of same type and obtained in same manner as the data for the test food product determined in steps (1) and (5) and wherein the calibration samples have known *in vivo* glycemic index values.

The present invention also provides an *in vitro* method for determining glycemic index for a test food product, said method comprising

(1) determining protein content and fat content of the test food product;
(2) providing the test food product in an essentially homogeneous and finely divided state to provide a test food sample wherein the test food product is ground at essentially liquid nitrogen temperatures;
(3) digesting an amount of the test food sample in the essentially homogeneous and finely divided state sufficient to provide a standardized amount of available carbohydrate with a mixture of digestive enzymes for a fixed period of time to provide a digested sample;
(4) treating the digested sample in its entirety immediately upon the fixed period of time to stop enzymatic reactions;
(5) determining amounts of glucose and at least two sugars or sugar alcohols selected from the group of fructose, galactose, lactose, sucrose, and maltitol in the digestive sample from step (4); and
(6) calculating the glycemic index of the test food product from data obtained in steps (1) and (5) for the test food product using a predictive equation or a predictive technique derived from multivariate analysis of calibration data for calibration samples wherein the calibration data is of same type and obtained in same manner as the data for the test food product determined in steps (1) and (5) and wherein the calibration samples have known *in vivo* glycemic index values.

The present *in vitro* method can be used to determine glycemic index values of solid, semi-solid, and liquid food products with significantly improved accuracy and precision as compared to currently available *in vitro* methods. Indeed, the accuracy and precision obtainable by the current inventive method appears to be comparable to commercially available *in vivo* methods but with significant savings in both time and costs.

For purposes of this invention, "essentially homogeneous and finely divided state" is intended to mean a degree of grinding equivalent to grinding the food item at temperatures sufficiently low such that the food item is a brittle solid. Generally, temperatures lower than about -40°C, temperatures preferably below about -78°C, and more preferably temperatures at about temperatures obtainable using liquid nitrogen (-196°C) are sufficient for grinding most food items which are solid or semi-solid at ambient temperatures. It is also generally preferred that ambient temperature solid materials are broken up or coarsely ground before such low temperature grinding. In one preferred embodiment, and especially for solid or semi-solid materials at ambient temperatures, grinding is accomplished using a liquid nitrogen-cooled grinding mill at or close to liquid nitrogen temperature. In many cases, liquid food products will not require any grinding since they are essentially homogeneous and the components are soluble in the carrier; if desired, however, such liquid food products may be also be ground at lower temperatures. Semi-solid materials may or may not require such low temperature grinding to obtain an essentially homogeneous and finely divided state. Thus, for example, a plain yogurt need not be further ground whereas a yogurt with fruit or other solid ingredients therein preferably is ground at low temperatures to obtain an essentially homogeneous and finely divided state. Further guidance on the required "homogeneous and finely divided state" can be found in the examples included with this specification.

Preferably the amounts of glucose and at least two sugars or sugar alcohols selected from the group of fructose, galactose, lactose, sucrose, and maltitol in the digestive sample from step (4) are determined using high performance liquid chromatography (HPLC) or equivalent quantitative methods. Preferably high performance ion chromatography (HPIC) is used to determine the amounts of sugars and/or sugar alcohols released during the treatment with enzymes. Even more preferably, the amounts of glucose, fructose, galactose, lactose, sucrose, and maltitol in the digestive sample from step (4) are determined using high performance ion chromatography (HPIC). Of course, for particular food samples, the digestive sample may be determined to be essentially zero for one or more of these sugars or sugar alcohols (i.e., particular sugars or sugar alcohols may be absent). In that case, the term or terms associated with the particular sugars and/or sugar alcohols will be zero (or close to zero) and the contribution to the GI for those sugars and/or sugar alcohols will be minimal.

Currently maltitol appears to be the only sugar alcohol that has been shown in the scientific literature to have an appreciable effect on blood sugar concentrations at levels normally encountered in food products. Since HPIC with the appropriate column is capable of determining many more sugars and sugar alcohols (not just maltitol), more sugars and sugar alcohols may be included in the analysis and calibration step if so desired.

An amount of the test food sample in the essentially homogeneous and finely divided state sufficient to provide a standardized amount of carbohydrate is used in the digestive step with a mixture of digestive enzymes for a fixed period of time to provide a digested sample. For purposes of this invention, the amount of the essentially homogeneous and finely divided test food sample used in the digestive step should contain a "standardized amount" of available carbohydrate. This standardized amount of available carbohydrate is selected so that sufficient sugars and/or sugar alcohols are obtained in the digestion step to allow for accurate determination of the sugar and sugar alcohols; moreover, this standardized amount of available carbohydrate allows the digestive enzymes to have similar amount of substrate to act upon. In the specific protocol illustrated in Example 1, it has generally been found that a standardized amount of available carbohydrates should be in the range of about 0.2 to about 1g, and more preferably about 0.5g. Higher or lower amounts can be used if sufficiently accurate determinations of the sugars and sugar alcohols can be obtained. Of course, once a standardized amount has been selected, that amount preferably is used for all determinations (including those used to calibrate the method and as well as, once calibrated, unknown samples). For the purpose of this method, available carbohydrate content is defined as the total carbohydrate content minus the dietary fiber content minus the total content of sugar alcohols other than maltitol. If additional sugar alcohols other than maltitol are to be included in the analysis and calibration, then the content of those sugar alcohols in the sample will need to be included as available carbohydrates as well.

Similarly, the fixed period for the digestive step is selected to provide sufficient sugars and/or sugar alcohols to allow for accurate determination of the sugar and sugar alcohols produced therein. In the specific protocol illustrated in Example 1, it has generally been found that fixed period should be in the range of about 15 to about 25 minutes, and more preferably about 20 minutes. Shorter or longer digestive times can be used if sufficient amounts of the sugar and/or sugar alcohols are produced to allow accurate determinations of the sugars and sugar alcohols. Of course, once a fixed time has been selected, that value should be used for all determinations (including those used to calibrate the method and as well as, once calibrated, unknown samples); in other words, a standardized digestive time should be used throughout all calibration and unknown sample determinations. Generally, efforts should be made to stop the digestive step within about $\pm$ 10 seconds of the selected fixed time, and even more preferably within about $\pm$ 5 seconds.

Figure 2 illustrates one embodiment of the present invention. The method begins with test food sample which may be solid, semi-solid, or liquid at ambient temperatures. As indicated above, the test food product should be rendered "essentially homogeneous and finely divided state." For most solid or semi-solid foods at ambient temperatures, this can be accomplished by grinding the food item at temperatures sufficiently low such that the food item is a brittle solid. Generally, temperatures lower than about -40°C, temperatures preferably below about -78°C, and more preferably temperatures at about temperatures obtainable using liquid nitrogen are sufficient for grinding most food items which are solid or semi-solid at ambient temperatures. It is also generally preferred that ambient temperature solid materials are broken up or coarsely ground before such low temperature grinding. Thus, as shown in Figure 2, the test food sample is preferably coarse ground to simply break the sample in to smaller particles and then finely ground to obtain the essentially homogeneous and finely divided sample. In one preferred embodiment, and especially for solid or semi-solid materials at ambient temperatures, fine grinding is accomplished using a liquid nitrogen-cooled grinding mill at or close to liquid nitrogen temperature. In many cases, liquid food products will not require any grinding since they are essentially homogeneous and the components are soluble in the carrier; if desired, however, such liquid food products may be also be ground at lower temperatures. Semi-solid materials may or may not require such low temperature grinding to obtain an essentially homogeneous and finely divided state. Thus, for example, a plain yogurt need not be further ground whereas a yogurt with fruit or other solid ingredients therein preferably is ground at low temperatures to obtain an essentially homogeneous and finely divided state.

As one skilled in the art will understand, it is not necessary, and indeed would be difficult, to specify the resulting particle size range of the "essentially homogeneous and finely divided state." Most food samples suitable for analysis by this method will contain significant moisture levels. Thus, even when ground at liquid nitrogen temperatures (where the solids will more easily be ground because of their brittle nature at such temperature), the particles may agglomerate when allowed to return to ambient temperature because of their water contents. It is the homogeneity and finely divided state obtainable with grinding at a low temperature, at least in part, which appears to allow the accuracy and precision of the present method. Thus, in most cases, the use of low-temperature grinding is the most appropriate means of producing a homogeneous, finely-divided mixture (especially with samples having a high degree of heterogeneity such as chocolate chip cookies, trail mixes, and the like). If however, the sample contains physically-hindered starch (such as that protected from digestive enzymes by intact or partially intact cell walls), it may be more appropriate to proceed directly from a coarse grinding or crushing to the digestion, since disrupting the cell structure may result in the starch becoming too accessible to the digestive enzymes, resulting in erroneously high GI numbers. Thus, samples like potato chips are preferably not ground at low temperature. When potato chips are ground to a fine powder under low temperatures, the resulting analysis yields a GI value of about 70-75. If potato chips are only coarsely ground (which is easy to effect since potato chips are fragile at ambient temperatures), the resulting analysis yields a GI value of about 55-60, which is more in line with the values in the literature (Am. J. Clin. Nutr., 76, 5-56 (2002) reports an an average value as $54\pm3$). Thus, as those skilled in the art will appreciate, the sample preparation technique is a balance of reducing sample heterogeneity without excessively disrupting native structures that may slow starch digestion.

Once the sample is in the desired homogeneous and finely divided state, it is subject to enzymatic digestion to mimic the digestive process. Pepsin is preferably added to begin hydrolysis of proteins. Then, a mixture of enzymes is used to digest the carbohydrates present in the sample to produce sugars and solubilize the free sugars and sugar alcohols that may be present in the sample. Importantly, standardized conditions are used when determining standards (both when the model coefficients are first determined and then as a check on the overall method) and unknowns. Although these standardized conditions may vary, once they are selected they should be maintained as closely as possible throughout the calibration of the method and then in the determination of unknown samples. Generally, the mixture of enzymes contains pancreatin, amyloglucosidase (AMG), and invertase in sufficient amounts to convert essentially all of the carbohydrates into sugars and solubilize any free sugars or sugar alcohols in the sample. Incubation with the enzyme mixture is generally carried out at 37°C for 20 minutes; once again, the exact temperature and time used for incubation (so long as they allow the enzymes to work) is less important than using the same incubation temperature and digestion time (i.e., fixed time for digestion) throughout the calibration process and unknown sample determinations.

Once the incubation has been carried out for the desired time (i.e., a standardized time of 20 minutes $\pm$ 10 seconds), the enzymatic reactions are immediately stopped. Preferably, ethanol is added to immediately stop the enzymatic reactions. The sample is preferably then filtered to remove any sediment or particulate matter which may be present and the sample collected. Importantly, from the time the incubation begins and until the sample is collected (i.e., after enzymatic reactions have been stopped), an integral or complete sample is maintained. That is, no separation or division of the sample are made during this time (except for the filtration after the enzymatic reactions are stopped). Maintaining the physical integrity of sample during this operation avoids errors associated with sampling what may be a non-homogeneous mixture. Thus, for example, the Englyst method may introduce significant sampling errors when an aliquot is taken from the digestion mixture after 20 minutes digestion since at that time the mixture is generally in the form of a suspension. The present method avoids such problems.

Once the final sample from the digestion mixture is obtained (preferably after filtration to remove any solid materials remaining), the sugar and sugar alcohol contents are measured using high performance liquid chromatography (HPLC) or equivalent quantitative methods. Preferably high performance ion chromatography (HPIC) is used since this technique generally gives superior separation of the sugars, especially glucose and galactose, allows the determination of both sugars and sugar alcohols (e.g., maltitol) in the same separation, and is generally less prone to interferences due to the highly selective nature of pulsed electrochemical detection. Suitable equipment and operating conditions are illustrated in Example 1. Once again, as those skilled in the art will realize, the specific equipment and operating parameters are less important than determining standardized conditions and then maintaining them throughout. Preferably the amounts of glucose and at least two sugars or sugar alcohols selected from the group of fructose, galactose, lactose, sucrose, and maltitol in the digestive sample are determined. Even more preferably, the amounts of glucose, fructose, galactose, lactose, sucrose, and maltitol in the digestive sample are determined. As noted above, additional sugars and sugar alcohols can be included in the analysis and calibration steps if desired.

Additionally the protein and fat contents for the test food sample are determined. These parameters can be determined using standard and conventional analytical techniques and/or can be calculated based on the ingredients of the test food sample. The determination of a predicted value for glycemic index is carried out using multivarient analysis such as, for example, a multiple linear regression (MLR), partial least squares (PLS), or neural net curve fitting procedures. The neural net curve fitting procedure appears to give the best fit to the data generated in the Examples and thus is generally preferred (see scatter plot in Figure 4A). The digestion mix is analyzed via the above HPIC procedure to produce quantitative values for glucose, fructose, galactose, lactose, sucrose, and maltitol content in the final sample digest. Those values, together with the total % protein and % fat of the original sample are preferably entered to a suitable statistical software program (e.g., JMP Statistical Software), which then predicts the GI values of the original sample based on the appropriate prediction or correlation equation or predictive technique.

The prediction equation or predictive technique is derived from the data for a calibration set for which the clinical or *in vivo* GI values are known. This is illustrated in Figure 2 wherein broken arrows are used to indicate the determination of the parameters for the prediction equation or predictive technique. The data is determined using the same standardized conditions and procedures as detailed above and in Example 1. The calibration set used in Example 1 includes food products and a series of pure standards for which the clinical GI values are known. The calibration standards used in Example 1 span the range of GI values from 19 to 100 and are treated in exactly the same manner as the other food samples in the calibration. All calibration samples and standards, and later all unknown samples, should be analyzed using the same standardized conditions and procedures. Using appropriate software, the data from the calibration samples and standards is used to derive an equation or other relationship which best fits the correlation between the clinical *in vivo* GI values and the prediction variables. Preferably, a multivariate analysis technique (e.g., MLR, PLS, or neural net) is used. Additional *in vivo* data from newly available calibration samples or standards can be, if desired, incorporated into the model to update and improve the prediction variables of the equation or predictive technique.

The method described herein preferably includes all the sugars listed above plus maltitol in the calibration. If, however, the samples likely to be encountered are known to not contain some of the components, they may be left out of the calibration (e.g., maltitol, galactose). Likewise, additional parameters or measurements associated with the food product (e.g., organic acid content, β-glucan content, and the like) can be included. The amount of sucrose in the digestive sample is typically found to be essentially zero since the invertase is expected to convert any sucrose present into glucose and fructose. If desired, the determination of sucrose can be used as an internal check. Thus, if the method is carried out properly the level of sucrose should be essentially zero; if significant amounts of sucrose are detected, the analysis is suspect and the sample should be repeated. Of course, this method of using sucrose as an effective internal standard should only be used if the standardized conditions and parameters selected for the method do, indeed, result in essentially no sucrose in the digestive sample. If the sample contains any substance which may inhibit invertase activity, the ability to quantitate the sucrose remaining may be useful in developing a calibration data set which takes that inhibitory effect into account in the calculation of the *in vitro* GI value.

The following examples are intended to illustrate the invention and not to limit it. Unless noted otherwise, all percentages and ratios are by weight.

Example 1. This example provides a detailed analytical protocol for carrying out the *in vitro* glycemic index determination of the present invention for both the calibration of the method and determination of unknown samples. In all cases where specific equipment, reagents, and/or specific procedures and operating parameters are suggested, it will be understood that equivalent equipment, reagents, and/or specific procedures and operating parameters can be used.

Apparatus:

1. Temperature-controlled shaking water bath (capable of about 175 strokes/min);
2. Vortex mixer;
3. Analytical balance (accurate to within $\pm$ 0.0001g);
4. Volumetric flasks, Class A, 100ml, 1000ml;
5. Pipets, Class A, 1ml, 2ml, 5ml, 6ml, 10ml;
6. Glass balls, 6 mm (Fisher #11-312-D or equivalent);
7. Glass sample vials (40 ml) with screw caps (VWR #66014-389 or equivalent);
8. Plastic centrifuge tubes (50 ml) with screw caps (VWR #21008-730 or equivalent);
9. Centrifuge;
10. Spex CertiPrep 6850 freezer mill or equivalent;
11. 1 Qt. wide-mouth Mason jars;
12. Filter funnels;
13. Whatman #4 fast filter paper, 12.5 cm circles, or equivalent;
14. Ring stands;
15. Anotop 10 Plus 0.2 micron 10mm diameter syringe filters;
16. HPIC equipment and reagents listed in section entitled "HPIC Procedure" below; and
17. JMP Statistical Software, version 5.1, SAS Institute, or equivalent.

Reagents:

1. 0.5 N Hydrochloric acid (HCl) (VWR #VW3201-1);
2. Sodium acetate (NaAc) (Acros #42425-5000);
3. Ethyl alcohol USP, 190 proof - 95% (95% EtOH) (Aaper Alcohol & Chemical Co.);

4. Invertase from bakers yeast, 215 U/mg solid (Sigma #I-9274);

5. Amyloglucosidase (AMG), 300 U/ml (Novozymes #AMG 300 L) (note: should be stored at 0-25°C and used within 3 months from date of delivery);

6. Pepsin from porcine stomach mucosa, 439 U/mg solid (Sigma #P-7000)

7. Guar gum (Sigma #G4129-250G);

8. Pancreatin from porcine pancreas (Sigma #P-7545);

9. Deionized water ($H_2O$); and

10. Liquid nitrogen; and

11. Pure standards of anhydrous glucose, fructose, sucrose, lactose, galactose, and maltitol (Minimum 99% purity, Sigma Aldrich Company or equivalent; should be stored in desiccator when not in use).

Solutions:

1. 0.05N HCl solution (dilute 100 ml 0.5N HCl to 1000ml with deionized water);

2. Pepsin/Guar Gum Solution (2195 U/ml pepsin and 0.5% guar gum (W/V) in 0.05 N HCl) - dissolve 0.5 g pepsin and 0.5 g guar gum in 100 ml 0.05 N HCl;

3. Invertase Stock Solution (510.7 U/ml in H2O) - dissolve 100 mg invertase in 42.10 ml $H_2O$;

4. 0.5 M Sodium Acetate Solution (0.5 M NaAc in $H_2O$) - dissolve 4.10 g sodium acetate in 100 ml $H_2O$;

5. 66% Ethyl Alcohol Solution (66% EtOH) (V/V) - mix 347 ml ethyl alcohol with 153 m; deionized water in a 1 quart mason jar and cap; this is enough solution for one sample; and

6. Enzyme Solution (recipe for one sample - 136 mg/ml pancreatin, 13.4 U/ml AMG and 25.43 U/ml invertase):

i. Weigh 1.0 g pancreatin into a 50 ml centrifuge tube;

ii. Add 6.67 ml $H_2O$ and vortex mix for 10 min to dissolve thoroughly (a stir bar or glass balls may be needed if preparing solution for use with for multiple samples);

iii. Centrifuge at 2000 rpm for 10 min; and

iv. For each test sample, transfer 6 ml supernatant to another 50 ml centrifuge tube, add 296 μl AMG and 330 μl Invertase Stock Solution.

Sample Preparation for Solid Sample:

1. Sample is placed in Waring Laboratory Micronizer and ground until sample is well mixed (generally 30 to 60 seconds is sufficient).

2. A 3 ounce/100g sub-sample from the Micronizer is then cryogenically ground in the Spex CertiPrep 6850 Freezer/Mill. The Spex CertiPrep 6850 Freezer/Mill is an impact grinder cooled by liquid nitrogen. The sample, embrittled by cold, is pulverized by the hammering of the impactor against the end plugs of the vial. The sample should occupy between about one third to about one half the height of the grinding vial (#6801).

3. The 6850 Freezer/Mill can be programmed using its keyboard. Control Modes and Conditions: Cycle 1 - Grinding Time 1.5 min; Rate (impact frequency) 10 cycles per second; and Pre-cooling Time 4 min. (i.e., time before grinding starts).

4. At the conclusion of the grinding cycle, empty the vial into a suitable container and cap.

Digestion Procedure:

1. Weigh an amount of sample equivalent to 0.50g available carbohydrate (available carbohydrate = total carbohydrates minus fiber minus sugar alcohols other than maltitol) into a 40 ml glass screw cap vial, add 5 ml $H_2O$, 10 ml freshly prepared pepsin/guar gum solution and 5 glass balls. A sample blank consisting of 0.5g of water is also run with each batch of samples.

2. Vortex mix the capped vial vigorously and place horizontally in a 37°C shaking water bath (175 strokes/min); shake for 30 min to allow hydrolysis of proteins by pepsin.

3. Add 5.0 ml 0.5 M NaAc (equilibrated to 37°C) to the vial.

4. Mix, then add 5 ml enyzme solution. Mix by inversion. The pH of this digestion mixture should be about 5.

5. Immediately place the original sample horizontally into the 37°C shaking water bath to continue the enzyme reaction. Shake for 20 minutes. Remove sample vial from shaker after 20 minutes ($\pm$ 10 seconds).

6. **Immediately** pour the contents of the vial into a mason jar containing 500 ml 66% EtOH to stop the reactions. Rinse the vial with several drops of EtOH solution from mason jar, cap the mason jar, then mix once by inversion. Immediately filter a portion of the contents of the mason jar through fast filter paper into a 40ml screw cap vial, cap, and save for HPIC analysis.

The digested sample is then analyzed for sugar and sugar alcohol content by HPIC as detailed below:

1. Equipment - Dionex chromatography system consisting of:

High-Performance Pump;
Liquid Chromatography Module;
Pulsed Electrochemical Detector;
Eluent Degas Module; and
Dionex Chromeleon Chromatography Workstation.

2. Reagents and Standards
Deionized water, 18 MΩ-cm resistance;
Sodium hydroxide solution, 50% w/w, low carbonate; and
Galactose, glucose, fructose, sucrose, lactose, and maltitol pure standards.

3. Conditions:

Columns: CarboPac™ PA1 analytical (4 x 250 mm) and AminoTrap guard (4 x 50 mm) columns;
Operating Conditions: Pressure: 1200-1500 psi; Inj. Volume: 10 μL; Eluent: 21 mM Sodium hydroxide (may be adjusted to achieve the best separation with each column; Note: CarboPac PA1 should be washed with 200 mM NaOH between runs to prevent carbonate from building up on the column); Flow Rate: 1 mL /min; and Detection: Pulsed amperometry, disposable gold working electrode and standard carbohydrate setting.

4. Preparation of Solutions:

a) Eluent: dilute 1.6725 g of NaOH solution (50% w/w, low carbonate) in 1.0 L of deionized water (18 MΩ-cm resistance);
b) Column Washing Solution: 200 mM NaOH: dilute 16 g of NaOH solution (50% w/w, low carbonate) in 1.0 L of deionized water (18 MΩ-cm resistance);

5. Preparation of Standards:

Accurately weigh about 0.1000 gm each of above 6 standards (i.e., galactose, glucose, fructose, sucrose, lactose, and maltitol pure standards) into a 100 ml volumetric flask to make a 1000 ppm mixed standard with deionized water (18 MΩ-cm resistance). Dilute this solution to produce the following working standards:

a) 1 ml diluted to 1000 ml including 66 ml ethyl alcohol (195 proof) to make 1 ppm standard;
b) 2 ml diluted to 100 ml including 6.6 ml ethyl alcohol (195 proof) to make 20 ppm standard;
c) 5 ml diluted to 100 ml including 6.6 ml ethyl alcohol (195 proof) to make 50 ppm standard; and
d) 10 ml diluted to 100 ml including 6.6 ml ethyl alcohol (195 proof) to make 100 ppm standard.

6. Sample Preparation and Injection:

The final digest solution from the digestion procedure is diluted 1:10 in deionized water, then filtered through a 0.2 micron Anotop10 Plus syringe filter into an autosampler vial. A sample of the solution (10 μl) is then injected into the chromatograph.

7. Chromatography Gradient Conditions:

| TIME (min) | Solvent (%) | | Comments |
|---|---|---|---|
| | 200 mM NaOH | 21 mM NaOH | |
| -20 | 100 | 0 | Regenerate Column |
| -9 | 0 | 100 | Re-equilibrate Column |
| 0 | 0 | 100 | Sample Injection |
| 36 | 0 | 100 | Completed |

8. Electrochemical Detector Parameters: Use preloaded waveform with Ag/AgCl reference electrode:

| Time | Potential | Integration |
|------|-----------|-------------|
| 0.0 | +0.10 | |
| 0.20 | +0.10 | Begin |
| 0.40 | +0.10 | End |
| 0.41 | -2.00 | |
| 0.42 | -2.00 | |
| 0.43 | -0.60 | |
| 0.44 | -0.10 | |
| 0.55 | -0.10 | |

Column storage solution: 21 mM Sodium hydroxide (NaOH)

9. Quantitative Determination of Sugars and Maltitol:

Mixed standards of 1, 20, 50, and 100 ppm are injected into the HPIC, and a calibration curve is constructed using the appropriate software for the chromatography system. If the concentration of one or more of the analytes in the sample exceeds that of the highest standard, the calibration curve should be expanded to include standards with concentrations higher than those found in the most concentrated sample. The sample blanks or test samples are then injected into the HPIC and the concentrations of galactose, glucose, fructose, sucrose, lactose, and maltitol are calculated from the calibration curve using peak areas. Concentrations from the calibration curve are multiplied by 10 to account for the 10-fold dilution of the samples and sample blank in step 6 above, and reported in terms of % of each analyte in the final digest.

The concentration values (%) for each analyte in the sample blank should be subtracted from those of each analyte in each sample to arrive at the blank-corrected sample concentration values (%).

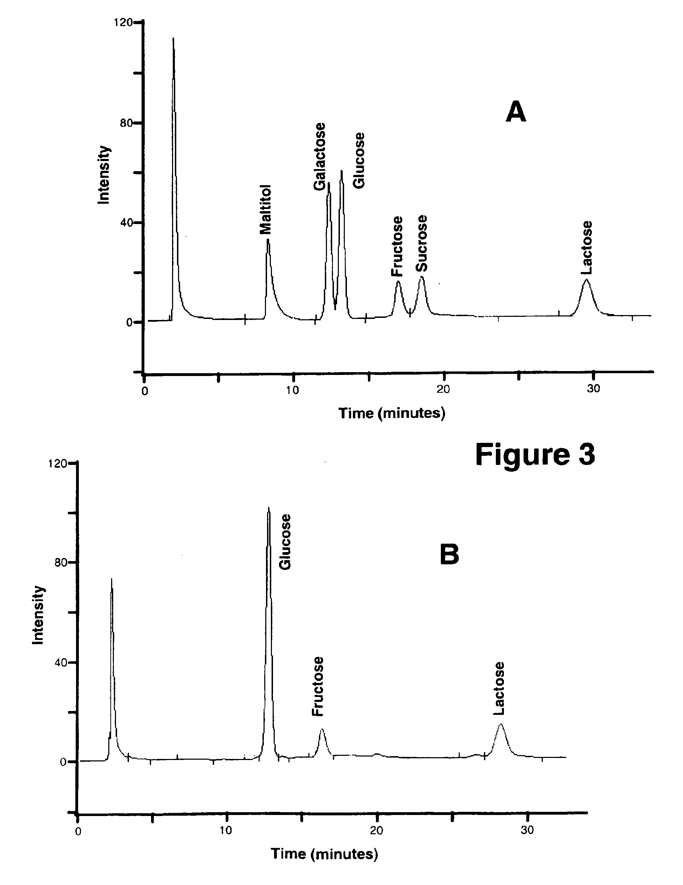

Example chromatograms of mixed standard (Panel A) and a typical unknown or test sample (Panel B) are shown in Figure 3.

Calculations:

The determination of a predicted value for glycemic index is accomplished via a multiple linear regression (MLR), partial least squares (PLS) or neural net curve fitting procedure. The digestion mix is analyzed via the above HPIC procedure to produce blank-corrected quantitative values for glucose, fructose, galactose, lactose, sucrose, and maltitol content in the final sample digest. Those values, together with the total % protein and % fat of the original sample, determined by analysis or calculation from ingredient values, are entered into a JMP Statistical Software spreadsheet, which then predicts the GI values of the original sample based on a prediction equation or predictive technique as appropriate for the specific curve fitting procedure used.

The prediction equation predictive technique is derived from the data for a calibration set for which the clinical *in vivo* GI values are known. The calibration set includes food products and a series of pure standards for which the clinical GI values are known. These standards used to calibrate the method in Example 2 span the range of GI values from 19 to 100 and are treated in exactly the same manner as the other food samples in the calibration. After they are analyzed, additional samples and/or standards may be added to the calibration set and the model parameters recalculated if the *in vivo* GI values are known for those samples and/or standards; such additions should strengthen the calibration by making the calibration set more broadly representative of the population of food samples for which GI may be determined. Thus, the prediction equation or predictive technique can be updated to include newly available or improved *in vivo* data. All calibration samples and standards were previously analyzed via the procedure described above, and the JMP Statistical Software was then used to fit an equation to the data which best fits the relationships between the clinical GI values and the prediction variables, using either the MLR, PLS, or neural net methods.

The method described herein includes all the sugars plus maltitol in the calibration. If, however, the samples likely to be encountered are known to not contain some of the components, they may be left out of the calibration (e.g., maltitol, galactose). The amount of sucrose in the digested sample is typically found to be essentially zero, due to the action of invertase, which converts any sucrose present into glucose and fructose. If, however, the sample contains an invertase activity inhibitor, the ability to include sucrose levels may be useful in developing calibration equations which takes that inhibitory effect into account in the calculation of the *in vitro* GI value. Maltitol is the only sugar alcohol that has been shown in the scientific literature to have an appreciable effect on blood sugar concentrations at levels normally encountered in food products. Since HPIC with the appropriate column is capable of determining many more sugars and sugar alcohols (not just maltitol), more sugars and sugar alcohols may be included in the analysis and calibration step if so desired.

Unknown samples are predicted by entering the % protein in the sample, % fat in the sample, and the blank-corrected concentration values for % glucose in the digest, % fructose in the digest, % lactose in the digest, % sucrose in the digest, % galactose in the digest, and % maltitol in the digest into the appropriate statistical software package (e.g., JMP Statistical Software), which then automatically predicts the GI value.

Example 2. A large number of commercially available food products and other food samples were examined using both *in vivo* and *in vitro* test protocols. Most (25 out of a total of 34) *in vivo* glycemic index values were determined using *in vivo* tests conducted by a well-known, well-respected private testing lab experienced in such testing; the remainder of such *in vivo* glycemic index values were taken from the scientific literature. These same samples were also examined using the *in vitro* method of the present invention as described in Example 1. These data were then used to calculate model parameters for the *in vitro* glycemic index test described herein using the MLR, PLS, and neural net methods available in JMP Statistical Software.

After the parent application was filed, it was discovered that the HCl solution used to prepare the Pepsin/Guar Gum Solution was 0.5 N rather than the desired 0.05 N. Since the Pepsin/Guar Gum Solution is used to prepare the enzyme digestion solution, the pH during enzyme digestion was about 1.1 rather than the desired about 5. This lower pH value is non-optimal for the enzyme digestion reaction. Thus, since the pH was too low, different levels of the various sugars were released relative to the fat and protein levels in the samples. However, since all samples were treated in the same manner (i.e., at the same pH during digestion), the relative values of the predicted glycemic index values remain valid. Of course, the model parameters and/or coefficients derived from this data set, would only be suitable for use if the lower pH during enzyme digestion was also used for new unknown samples. Examples 3 and 4 below were carried out using the proper HCl solution; thus the enzyme digestion reactions were carried out under more optimal pH conditions.

The following model parameters were obtained using the MLR method:

| Term | Value | Std Error | t Ratio | Prob>ltl |
|---|---|---|---|---|
| Intercept | 63.080214 | 6.498483 | 9.71 | <0.0001 |
| Protein (%) | -0.974313 | 0.141985 | -6.86 | <0.0001 |
| Fat (%) | -0.67442 | 0.087795 | -7.68 | <0.0001 |
| Glucose (%) | 364.97478 | 75.57708 | 4.83 | <0.0001 |
| Fructose (%) | -452.5341 | 75.17166 | -6.02 | <0.0001 |
| Lactose (%) | -191.8138 | 84.17182 | -2.28 | 0.0311 |
| Galactose (%) | -437.3615 | 90.44204 | -4.84 | <0.0001 |
| Maltitol (%) | -298.0102 | 92.58933 | -3.22 | 0.0034 |

Thus, the equation used to estimate the *in vitro* glycemic index using the MLR method would be:

$$GI = 63.080214 - 0.974313\,Protein(\%) - 0.67442\,Fat(\%) + 367.97478\,Glucose(\%)$$
$$- 452.5341\,Fructose(\%) - 191.8138\,Lactose(\%) - 437.3615\,Galactose(\%)$$
$$- 298.0102\,Maltitol(\%).$$

The relevant statistical parameters for this model were as follows:
Summary of Fit:

| | |
|---|---|
| $R^2$ | 0.948355 |
| $R^2$ Adj | 0.934451 |
| Root Mean Square Error | 6.094049 |
| Mean of Response | 46.52941 |
| Observations (or Sum Wgts) | 34 |

Analysis of Variance:

| Source | DF | Sum of Squares | Mean Square | F Ratio |
|---|---|---|---|---|
| Model | 7 | 17730.897 | 2532.99 | 68.2057 |
| Error | 26 | 965.573 | 37.14 | Prob > F |
| C. Total | 33 | 18696.471 | | <.0001 |

The following model parameters were obtained using the PLS method with five latent variables:

| Term | Value |
|---|---|
| Intercept | 62.745005 |
| Protein (%) | -0.986004 |
| Fat (%) | -0.670993 |
| Glucose (%) | 369.91833 |
| Fructose (%) | -446.4468 |
| Lactose (%) | -195.07 |
| Galactose (%) | -425.7489 |
| Maltitol (%) | -291.9779 |

Thus, the equation used to estimate the *in vitro* glycemic index using the PLS method would be:

$$GI = 62.745005 - 0.986004 \text{Protein}(\%) - 0.670993 \text{Fat}(\%) + 369.91833 \text{Glucose}(\%)$$
$$- 446.4468 \text{Fructose}(\%) - 195.07 \text{Lactose}(\%) - 425.7489 \text{Galactose}(\%)$$
$$- 291.9779 \text{Maltitol}(\%).$$

The relevant statistical parameters for this model were as follows:
Summary of Fit:

| | |
|---|---|
| $R^2$ | 0.939819 |
| $R^2$ Adj | 0.937938 |
| Root Mean Square Error | 5.929726 |
| Mean of Response | 46.52941 |
| Observations (or Sum Wgts) | 34 |

The following model parameter weights were obtained using the neural net method:
**Neural Net Table I:**

| Parameter Weight | Value |
|---|---|
| H1:Intercept | 0.6715187765 |
| H1:Protein (%) | 0.3364859726 |
| H1:Fat (%) | 0.2031260198 |
| H1:Glucose (%) | -0.697405591 |

(continued)

| Parameter Weight | Value |
|---|---|
| H1:Fructose (%) | 0.0085016679 |
| H1:Lactose (%) | -0.162454793 |
| H1:Galactose (%) | 0.0632785954 |
| H1:Maltitol (%) | -0.053195992 |
| GI:Intercept | 3.994861093 |
| GI:H1 | -6.220038739 |

The neural net method does not provide a simple prediction equation. Rather, this method provides a series of parameter weights and a network topology which can then be used to obtain predicted results using a suitable software program (in this case the JMP Statistical Software). The fit obtained using this method is illustrated graphically in Figure 5A. The relevant parameters for this model were as follows:

| | Specify |
|---|---|
| Hidden Nodes | 1 |
| Overfit Penalty | 0.01 |
| Number of Tours | 200 |
| Max Iterations | 50 |
| Converge Criterion | 0.00001 |

Results:

| SSE | 4.1349837524 |
|---|---|
| Penalty | 0.3981397476 |
| Total | 4.5331235 |
| N | 65 |

| 200 | Converged At Best |
|---|---|
| 0 | Converged Worse Than Best |
| 0 | Stuck on Flat |
| 0 | Failed to Improve |
| 0 | Reached Max Iter |

| Y | SSE | SSE Scaled | RMSE | RMSE Scaled | $R^2$ |
|---|---|---|---|---|---|
| GI | 1540.3848224 | 4.1349837524 | 4.94475002 | 0.25619263 | 0.9354 |

The resulting model parameters were then used to calculate the *in vitro* glycemic index values using each of the data fitting methods. The results from the following commercially available products were used to calculate the correlation parameters. The resulting *in vitro* glycemic index values determined using each of the data fitting methods are compared to the *in vivo* glycemic index values:

| Sample | In vivo GI* | In vitro GI | | |
|---|---|---|---|---|
| | | PLS | MLR | Neural Net |
| Trail Mix Blend 1 | 16 | 13 | 13 | 19 |
| Trail Mix Blend 2 | 19 | 13 | 13 | 19 |
| Trail Mix Blend 3 | 20 | 21 | 21 | 21 |
| Trail Mix Blend 4 | 18 | 15 | 15 | 19 |
| Trail Mix Blend 5 | 25 | 16 | 16 | 20 |
| Trail Mix Blend 6 | 26 | 19 | 19 | 21 |
| Trail Mix Blend 7 | 35 | 37 | 37 | 30 |
| Fruit Filled Cookie 1 | 77 | 73 | 73 | 75 |
| Fruit Filled Cookie 2 | 63 | 66 | 66 | 67 |
| Peanut Brittle Bar | 38 | 34 | 34 | 28 |
| Saltine Cracker | 88 | 79 | 79 | 83 |
| Buttery Cracker | 67 | 66 | 66 | 64 |
| Chocolate Cookie | 50 | 58 | 58 | 55 |
| Baked Cracker Chip | 59 | 65 | 65 | 64 |
| Whole Wheat Cracker | 71 | 70 | 70 | 73 |
| Chocolate Chip Cookie | 45 | 52 | 52 | 45 |
| Cereal Blend | 61 | 52 | 52 | 55 |
| Cottage Cheese Mix Product | 51 | 52 | 53 | 52 |
| Potato Flakes 1 | 81 | 85 | 85 | 89 |
| Potato Flakes 2 | 89 | 83 | 84 | 88 |
| Energy Bar 1 | 31 | 36 | 36 | 34 |
| Energy Bar 2 | 36 | 42 | 42 | 39 |
| Energy Bar 3 | 27 | 38 | 38 | 35 |
| Energy Bar 4 | 40 | 39 | 40 | 37 |
| Coca-Cola | 63** | 59 | 59 | 58 |
| Whole Milk | 40** | 39 | 40 | 40 |
| Clear Unsweetened Apple Juice | 44** | 49 | 49 | 46 |
| Glucose | 100** | 99 | 99 | 96 |
| Sucrose | 68** | 64 | 64 | 65 |
| Fructose | 19** | 21 | 21 | 23 |
| Lactose | 46** | 44 | 45 | 46 |
| Maltitol | 35† | 35 | 34 | 35 |

(continued)

| Sample | In vivo GI* | In vitro GI | | |
|---|---|---|---|---|
| | | PLS | MLR | Neural Net |
| Galactose | 20†† | 21 | 20 | 20 |
| * In vivo values were experimentally determined using a private testing laboratory unless otherwise noted.<br>** In vivo values taken from: "International table of glycemic index and glycemic load values," Am. J. Clin. Nutr., 76, 5-56 (2002).<br>† In vivo values taken from: Livesy, Nutr. Res. Rev., 16(2), 163-91 (2003).<br>†† In vivo values taken from: Jentjens et al., Eur. J. App.I Physiol. 88, 459-465 (2003). | | | | |

A scatter plot of this data obtained using the neural net as curve fitting procedure is shown in Figure 4A. A $R^2$ value of 0.97 is obtained using this method. The standard deviation for the *in vitro* method (based on duplicate determinations of 34 total samples) was $\pm 1.08$ GI units or $\pm 2.33$ percent relative. Thus, this method appears to have very high precision and high predictive value.

Example 3. After filing the original application, work was continued and additional data points have been added and certain modifications made to the method to provide an even more accurate and precise *in vitro* method. As noted in Example 2 above, the pH during the enzyme digestion step in Example 2 was too low. The proper pH value for the enzyme digestion step should be about 5; other than that modification, the samples were treated in the same manner as in Example 2. Thus, 31 of the samples used in Example 2 were rerun to obtain data at the more optimal enzyme digestion pH. Additionally, 34 new samples have been added to the rerun sample from Example 2 to bring the total number of samples to 65. This *in vitro* method (current as of the date of this continuation-in-part application) is based on samples enzyme digested at the more optimal pH value and is presented herein. Of course, as one skilled in the art realizes, the accuracy and precision of this method is also likely to be increased even further as additional *in vivo* data for calibration samples and/or standards becomes available and is incorporated into the in vitro model during the calibration procedure.

The following model parameters were obtained using the MLR method:

| Term | Value | Std Error | t Ratio | Prob>ItI |
|---|---|---|---|---|
| Intercept | 26.779686 | 2.179648 | 12.29 | <0.0001 |
| Protein (%) | -0.967251 | 44.81633 | -8.92 | <0.0001 |
| Fat (%) | -0.385715 | 0.073903 | -5.22 | <0.0001 |
| Glucose (%) | 611.60013 | 30.00233 | 20.39 | <0.0001 |
| Lactose (%) | 301.95014 | 44.81633 | 6.74 | <0.0001 |
| Maltitol (%) | 169.03383 | 54.85795 | 3.08 | .0032 |
| (Glucose (%)-0.05718)*(Protein(%)-8.24967) | -18.16062 | 5.456618 | -3.33 | 0.0015 |

Thus, the equation used to estimate the *in vitro* glycemic index using the MLR method and the expanded data set would be:

$$GI = 26.779686 - 0.967251 Protein(\%) - 0.385715 Fat(\%) + 611.60013 Glucose(\%)$$
$$+ 301.95014 Lactose(\%) + 169.03383 Maltitol(\%)$$
$$- 18.16062 [Glucose (\%)-0.05718]*[Protein (\%)-8.24967].$$

The relevant statistical parameters for this model were as follows:
**Summary of Fit:**

| $R^2$ | 0.940178 |
|---|---|

(continued)

| R² Adj | 0.93399 |
|---|---|
| Root Mean Square Error | 4.958869 |
| Mean of Response | 50.4 |
| Observations (or Sum Wgts) | 65 |

**Analysis of Variance:**

| Source | DF | Sum of Squares | Mean Square | F Ratio |
|---|---|---|---|---|
| Model | 6 | 22415.358 | 2532.99 | 151.9250 |
| Error | 58 | 1426.242 | 24.59 | Prob > F |
| C. Total | 64 | 23841.6000 | | <.0001 |

The following model parameters were obtained using the PLS method with five latent variables:

| Term | Value |
|---|---|
| Intercept | 26.264529 |
| Protein (%) | -1.048186 |
| Fat (%) | -0.248138 |
| Glucose (%) | 621.7824 |
| Fructose (%) | -52.7993 |
| Lactose (%) | 233.67679 |
| Galactose (%) | -61.21071 |
| Maltitol (%) | 84.689245 |

Thus, the equation used to estimate the *in vitro* glycemic index using the PLS method would be:

$$GI = 26.264529 - 1.048186 \text{Protein}(\%) - 0.248138 \text{Fat}(\%) + 621.7824 \text{Glucose}(\%)$$
$$- 52.7993 \text{Fructose}(\%) + 233.67679 \text{Lactose}(\%) - 61.21071 \text{Galactose}(\%)$$
$$+ 84.689245 \text{Maltitol}(\%).$$

The relevant statistical parameters for this model were as follows:

**Summary of Fit:**

| R² | 0.930041 |
|---|---|
| R² Adj | 0.9289831 |
| Root Mean Square Error | 5.145383 |
| Mean of Response | 50.4 |
| Observations (or Sum Wgts) | 65 |

The following model parameter weights were obtained using the neural net method:

**Neural Net Table I:**

| Parameter Weight | Value |
|---|---|
| H1:Intercept | 0.6715187765 |
| H1:Protein (%) | 0.3364859726 |
| H1:Fat (%) | 0.2031260198 |
| H1:Glucose (%) | -0.697405591 |
| H1:Fructose (%) | 0.0085016679 |
| H1:Lactose (%) | -0.162454793 |
| H1:Galactose (%) | 0.0632785954 |
| H1:Maltitol (%) | -0.053195992 |
| GI:Intercept | 3.994861093 |
| GI:H1 | -6.220038739 |

As noted in Example 2, the neural net method does not provide a simple prediction equation. Rather, this method provides a series of parameter weights and a network topology which can then be used to obtain predicted results using a suitable software program (in this case the JMP Statistical Software). The fit obtained using this method is illustrated graphically in Figure 4A. The relevant parameters for this model were as follows:

| | Specify |
|---|---|
| Hidden Nodes | 1 |
| Overfit Penalty | 0.01 |
| Number of Tours | 200 |
| Max Iterations | 50 |
| Converge Criterion | 0.00001 |

**Results:**

| SSE | 4.1349837524 |
|---|---|
| Penalty | 0.3981397476 |
| Total | 4.5331235 |
| N | 65 |

| 200 | Converged At Best |
|---|---|
| 0 | Converged Worse Than Best |
| 0 | Stuck on Flat |
| 0 | Failed to Improve |
| 0 | Reached Max Iter |

| Y | SSE | SSE Scaled | RMSE | $R^2$ |
|---|---|---|---|---|
| GI | 1540.3848224 | 4.1349837524 | 4.94475002 | 0.9354 |

The resulting model parameters were then used to calculate the *in vitro* glycemic index values using each of the data fitting methods as in Example 2. The results from the following products, about 33 more than in Example 2, were used to calculate correlation parameters for each model. The resulting *in vitro* glycemic index values determined using each of the data fitting models or methods with the expanded data set are compared to the *in vivo* glycemic index values:

| Sample | *In-vivo* GI* | *In-vitro* GI | | |
|---|---|---|---|---|
| | | PLS | MLR | NN |
| Trail Mix Blend 1 | 16 | 18 | 20 | 21 |
| Trail Mix Blend 2 | 19 | 17 | 20 | 20 |
| Trail Mix Blend 3 | 20 | 27 | 24 | 25 |
| Trail Mix Blend 4 | 18 | 21 | 21 | 22 |
| Trail Mix Blend 5 | 25 | 21 | 22 | 22 |
| Trail Mix Blend 6 | 26 | 22 | 23 | 23 |
| Trail Mix Blend 7 | 35 | 38 | 38 | 35 |
| Peanuts | 14** | 6 | 10 | 16 |
| Fruit Filled Cookie 1 | 77 | 69 | 71 | 70 |
| Fruit Filled Cookie 2 | 63 | 64 | 67 | 65 |
| Peanut Brittle Bar | 38 | 32 | 31 | 29 |
| Saltine Cracker *** | 74** | 69 | 67 | 69 |
| Buttery Cracker | 67 | 69 | 67 | 67 |
| Chocolate Cookie | 50 | 50 | 50 | 47 |
| Baked Cracker Chip | 59 | 68 | 68 | 67 |
| Whole Wheat Cracker | 71 | 68 | 66 | 67 |
| Chocolate Chip Cookie | 45 | 49 | 47 | 44 |
| Breakfast Cereal 1 | 63‡‡ | 58 | 59 | 57 |
| Breakfast Cereal 2 | 47‡‡ | 50 | 49 | 49 |
| Breakfast Cereal 3 | 61** | 61 | 63 | 61 |
| Breakfast Cereal 4 | 81** | 76 | 78 | 78 |
| Breakfast Cereal 5 | 74** | 68 | 68 | 69 |
| Multi-Grain Instant Oatmeal | 55‡‡ | 67 | 64 | 67 |
| Ice Cream, full fat, vanilla | 61** | 61 | 63 | 61 |
| Cottage Cheese Mix Product | 51 | 42 | 45 | 41 |
| Potato Flakes‡‡‡ | 85 | 76 | 76 | 78 |
| Baked Potato | 60** | 61 | 61 | 60 |
| Energy Bar 1 | 31 | 29 | 32 | 29 |
| Energy Bar 2 | 36 | 36 | 35 | 34 |
| Energy Bar 3 | 27 | 35 | 33 | 33 |
| Energy bar 4 | 40 | 35 | 34 | 33 |
| Coca-Cola**** | 58** | 51 | 53 | 51 |
| Whole Milk | 40** | 39 | 40 | 39 |
| Apple Juice | 44** | 44 | 44 | 44 |

(continued)

| Sample | In-vivo GI* | In-vitro GI | | |
|---|---|---|---|---|
| | | PLS | MLR | NN |
| Orange Juice 1 | 50** | 53 | 54 | 53 |
| Orange Juice 2 | 50** | 51 | 53 | 51 |
| Cranberry Juice Cocktail | 60** | 57 | 60 | 58 |
| Whole Wheat Hamburger Bun | 62‡‡ | 73 | 70 | 74 |
| Hot Dog Bun | 71** | 71 | 71 | 72 |
| White Bread | 71** | 70 | 71 | 71 |
| Whole Wheat Bread | 62** | 61 | 61 | 60 |
| Pretzels | 83** | 77 | 75 | 79 |
| Popcorn (Microwave, Butter Flavor) | 72 | 75 | 71 | 73 |
| Raisin Bran Flax Muffin | 52‡‡ | 53 | 55 | 52 |
| Rice, Brown, Long Grain | 55** | 62 | 62 | 61 |
| Rice, White, Long Grain | 69** | 68 | 70 | 69 |
| Rice and Corn Snack | 81‡‡ | 80 | 81 | 82 |
| Whole Wheat Pasta | 57‡‡ | 63 | 60 | 62 |
| Grapes | 46** | 49 | 49 | 48 |
| Orange | 48** | 45 | 45 | 44 |
| Peaches, Canned | 38** | 45 | 45 | 45 |
| Banana | 47** | 44 | 43 | 43 |
| Apple | 38** | 42 | 41 | 42 |
| Pear | 38** | 33 | 30 | 33 |
| Corn (Canned Yellow Sweet Kernels) | 46** | 46 | 45 | 44 |
| Carrots (Fresh Baby) | 47** | 43 | 41 | 41 |
| Vegetarian Chili | 36‡‡ | 38 | 40 | 36 |
| Tomato and Basil Pasta Sauce | 33‡‡ | 43 | 42 | 42 |
| Pasta Fagiola Soup | 52‡‡ | 64 | 66 | 64 |
| Glucose | 100** | 92 | 99 | 95 |
| Sucrose | 61‡ | 57 | 60 | 58 |
| Fructose | 19** | 21 | 18 | 26 |
| Lactose | 46** | 49 | 47 | 49 |
| Maltitol | 35† | 35 | 35 | 35 |

(continued)

| Sample | In-vivo GI* | In-vitro GI | | |
|---|---|---|---|---|
| | | PLS | MLR | NN |
| Galactose | 20†† | 20 | 18 | 20 |

*In vivo* values were experimentally determined using a private testing laboratory unless otherwise noted.

** *In vivo* values taken from: "International table of glycemic index and glycemic load values," Am. J. Clin. Nutr., 76, 5-56 (2002).

*** This *in vivo* value for Saltine Cracker differs from that used in Example 2. This present value appears to be more accurate.

**** This *in vivo* value, based on an average for two determinations, for Coca-Cola differs from that used in Example 2 which was based on only one determination. This present value appears to be more accurate.

‡ *In vivo* value taken from: "International table of glycemic index and glycemic load values," Am. J. Clin. Nutr., 76, 5-56 (2002) (outliers excluded). Thus, this value differs slightly from the one used in Example 2.

‡‡ *In vivo* values taken from manufacturer's data.

‡‡‡ This *in vivo* value for Potato Flakes is an average of Potato Flakes 1 and Potato Flakes 2 in Example 2. The two Potato Flake samples in Example 2 were determined to be very similar in nature.

† *In vivo* values taken from: Livesy, Nutr. Res. Rev., 16(2), 163-91 (2003).

†† *In vivo* values taken from: Jentjens et al., Eur. J. App. Physiol. 88, 459-465 (2003).

A scatter plot of this data obtained using the MLR curve fitting procedure is shown in Figure 5A. A $R^2$ value of 0.94 is obtained using this method. The standard deviation for the *in vitro* method (based on duplicate determinations of 65 total samples) was $\pm$ 4 GI units or $\pm$ 6 percent relative. Thus, this method appears to have very high precision and high predictive value.

The *in vitro* method can then be used to calculate the *in vitro* GI value for an unknown food product or food ingredient by simply carrying out the method described herein for the sample preparation and analysis and using the data and the desired predictive equation determined from the calibration data. Of course, as demonstrated by comparing Examples 2 and 3, the calibration results and predictive equation data will depend on the actual conditions under which the calibration data set and unknown samples are analyzed (especially as regard to the enzyme digestion step). However, as long as the same procedure is used for all samples, variations in the analysis procedure do not appear to significantly effect the predictive ability of the present method even when the predictive equations differ widely. This, we believe, demonstrates the robustness of this *in vitro* method. As the size of the calibration data set is increased, either in number of samples and/or accuracy of the *in vivo* GI values used, the accuracy and precision of the method is expected to increase.

As noted above, Garsetti et al. provided a scatter plot with an $R^2$ value of 0.25 for the *in vitro* Englyst method, thus indicating that this method had little predictive value. For further comparison purposes, the rapidly available glucose (RAG as expressed as grams glucose released in 20 minutes/100 g food) as obtained using the calculation techniques used in the Englyst method (i.e., only based on the glucose release after 20 minutes digestion) on the data from Examples 2 and 3 were separately correlated to *in vivo* GI values using the calculations and parameters described in the *in vitro* Englyst method (i.e., using only glucose released after 20 minutes) as detailed in Englyst et al., Brit. J. Nutr., 75, 327-337 (1996) (Figure 4B) and Englyst et al., Am. J. Clin. Nutr., 69, 448-454 (1999) (Figure 4C). Such scatter plots are shown in Figures 4B and 4C using the Example 2 data and in Figures 5B and 5C using the Example 4 data. In Figures 4B and 5B, the *in vivo* GI is plotted versus RAG/(% Available Carbohydrates) for the Example 2 and Example 3 data, respectively; in Figures 4C and 5C, the *in vivo* GI is plotted versus RAG for the Example 2 and Example 3 data, respectively. When such calculations were carried out using Example 2 data, the $R^2$ values of the Englyst et al. method increased to about 0.41 and about 0.68 in Figures 4B and 4C, respectively; using the Example 3 data, the $R^2$ values of the Englyst et al. method increased to about 0.56 and about 0.58 in Figures 5B and 5C, respectively. Although these represent improvements as compared to the Garsetti et al. scatter plot, the predictive value of the method is still poor.

We believe, as demonstrated by the $R^2$ value of about 0.94 to about 0.97 (depending on the specific curve fitting method used and the conditions under which the data is generated) for the present inventive method, that, for the first time, an *in vitro* method has been provided which can be used to accurately and precisely determine glycemic index values for a wide variety of food products and/or food ingredients. This method can be used to easily, quickly, and inexpensively obtain accurate glycemic index values which compare favorably in terms of accuracy to the much more time consuming and expensive human subject *in vivo* testing currently recommended. Moreover, the accuracy and precision of this method is likely to be increased even further as additional *in vivo* data for calibration samples and/or standards becomes available and is incorporated into the *in vitro* model during the calibration procedure. This advance in the art should increase the usefulness of glycemic index and related values (e.g., glycemic load values determined by multiplying the glycemic index by the amount of carbohydrate in grams provided by a food and dividing the total by 100) in human nutrition.

Example 4. The samples used in Example 2 (using a less than optimal pH value in the enzymatic reaction step) were rerun in Example 3 using a more optimal pH value. The data from Example 3 for only the rerun Example 2 data set (i.e., excluding the additional samples added in Example 3) was used to generate a new set of the various curve fitting parameters. This example - which is based on 31 of the 34 samples of Example 2 - effectively illustrates the *in vitro* analysis of Example 2 with the samples run at a more optimal pH value. This allows for a more direct comparison of the correlation coefficients or parameters from Example 2 and Example 3 where only the number of samples in each data set was varied.

The following model parameters for this modified data set were obtained using the MLR method:

| Term | Value | Std Error | t Ratio | Prob>ltl |
|---|---|---|---|---|
| Intercept | 30.502796 | 3.388751 | 9.00 | <0.0001 |
| Protein (%) | -0.787639 | 0.137003 | -5.75 | <0.0001 |
| Fat (%) | -0.515649 | 0.088565 | -5.82 | <0.0001 |
| Glucose (%) | 573.03525 | 51.23366 | 11.18 | <0.0001 |
| Lactose (%) | 275.61246 | 45.69519 | 6.03 | <0.0001 |
| Maltitol (%) | 147.62386 | 51.90435 | 2.84 | 0.0090 |
| (Glucose (%)-0.05091)*(% Protein-10.0429) | -20.39966 | 6.583621 | -3.10 | 0.0049 |

Thus, the equation used to estimate the *in vitro* glycemic index using the MLR method:

$$GI = 30.502796 - 0.787639\text{Protein}(\%) - 0.515649\text{Fat}(\%) + 573.03525\text{Glucose}(\%)$$
$$+ 275.61246\text{Lactose}(\%) + 147.62386\text{Maltitol}(\%)$$
$$- 20.39966[\text{Glucose }(\%)\text{-}0.05091]*[\text{ Protein }(\%)\text{-}10.0429].$$

The relevant statistical parameters for this model were as follows:

**Summary of Fit:**

| | |
|---|---|
| $R^2$ | 0.968035 |
| $R^2$ Adj | 0.960043 |
| Root Mean Square Error | 4.393218 |
| Mean of Response | 45.03226 |
| Observations (or Sum Wgts) | 31 |

**Analysis of Variance:**

| Source | DF | Sum of Squares | Mean Square | F Ratio |
|---|---|---|---|---|
| Model | 6 | 14027.759 | 2337.96 | 121.1355 |
| Error | 24 | 463.209 | 19.31 | Prob > F |
| C. Total | 30 | 14490.968 | | <.0001 |

The following model parameters were obtained using the PLS method with five latent variables:

| Term | Value |
|---|---|
| Intercept | 48.443999 |
| Protein (%) | -1.003763 |
| Fat (%) | -0.492915 |
| Glucose (%) | 443.60721 |
| Fructose (%) | -265.4383 |
| Lactose (%) | -33.78516 |
| Galactose (%) | -277.1671 |
| Maltitol (%) | -142.2773 |

Thus, the equation used to estimate the *in vitro* glycemic index using the PLS method would be:

$$GI = 48.443999 - 1.003763 \text{Protein}(\%) - 0.492915 \text{Fat}(\%) + 443.60721 \text{Glucose}(\%)$$
$$- 265.4383 \text{Fructose}(\%) - 33.78516 \text{Lactose}(\%) - 277.1671 \text{Galactose}(\%)$$
$$- 142.2773 \text{Maltitol}(\%).$$

The relevant statistical parameters for this model were as follows:
**Summary of Fit:**

| | |
|---|---|
| $R^2$ | 0.964188 |
| $R^2$ Adj | 0.962953 |
| Root Mean Square Error | 4.230216 |
| Mean of Response | 45.03226 |
| Observations (or Sum Wgts) | 31 |

The following model parameter weights were obtained using the neural net method:
**Neural Net Table I:**

| Parameter Weight | Value |
|---|---|
| H1:Intercept | 0.8572040599 |
| H1:Protein (%) | 0.3767226601 |
| H1:Fat (%) | 0.4495966298 |
| H1:Glucose (%) | -0.651892945 |
| H1:Fructose (%) | 0.2134796555 |
| H1:Lactose (%) | -0.042105783 |
| H1:Galactose (%) | 0.2610403756 |
| H1:Maltitol (%) | 0.0659243194 |
| GI:Intercept | 3.2202762866 |
| GI:H1 | -4.803405337 |

As noted in Examples 2 and 3, the neural net method does not provide a simple prediction equation. Rather, this method provides a series of parameter weights and a network topology which can then be used to obtain predicted results using a suitable software program (in this case the JMP Statistical Software). The relevant parameters for this model were as follows:

|  | Specify |
| --- | --- |
| Hidden Nodes | 1 |
| Overfit Penalty | 0.01 |
| Number of Tours | 200 |
| Max Iterations | 50 |
| Converge Criterion | 0.00001 |

**Results:**

| SSE | 1.027472638 |
| --- | --- |
| Penalty | 0.2469635769 |
| Total | 1.2744362149 |
| N | 31 |

| 200 | Converged At Best |
| --- | --- |
| 0 | Converged Worse Than Best |
| 0 | Stuck on Flat |
| 0 | Failed to Improve |
| 0 | Reached Max Iter |

| Y | SSE | SSE Scaled | RMSE | $R^2$ |
| --- | --- | --- | --- | --- |
| GI | 496.30242843 | 1.027472638 | 4.13689217 | 0.9658 |

The resulting model parameters were then used to calculate the *in vitro* glycemic index values using each of the data fitting methods. The results from the following commercially available products were used to calculate the correlation parameters. The resulting *in vitro* glycemic index values determined using each of the data fitting methods are compared to the *in vivo* glycemic index values:

| Sample* | *In vivo* GI** | *In vitro* GI | | |
| --- | --- | --- | --- | --- |
|  |  | **PLS** | **MLR** | **Neural Net** |
| Trail Mix Blend 1 | 16 | 19 | 19 | 21 |
| Trail Mix Blend 2 | 19 | 18 | 19 | 21 |
| Trail Mix Blend 3 | 20 | 19 | 22 | 21 |
| Trail Mix Blend 4 | 18 | 19 | 20 | 21 |
| Trail Mix Blend 5 | 25 | 20 | 21 | 22 |
| Trail Mix Blend 6 | 26 | 20 | 21 | 22 |
| Trail Mix Blend 7 | 35 | 37 | 38 | 33 |

(continued)

| Sample* | In vivo GI** | In vitro GI | | |
|---|---|---|---|---|
| | | PLS | MLR | Neural Net |
| Peanuts | 14** | 6 | 10 | 16 |
| Fruit Filled Cookie 1 | 77 | 71 | 73 | 72 |
| Fruit Filled Cookie 2 | 63 | 65 | 69 | 66 |
| Peanut Brittle Bar | 38 | 33 | 30 | 29 |
| Saltine Cracker | 74 | 73 | 69 | 74 |
| Buttery Cracker | 67 | 68 | 67 | 66 |
| Chocolate Cookie | 50 | 50 | 47 | 45 |
| Baked Cracker Chip | 59 | 70 | 69 | 70 |
| Whole Wheat Cracker | 71 | 72 | 67 | 73 |
| Chocolate Chip Cookie | 45 | 50 | 47 | 45 |
| Cottage Cheese Mix Product | 51 | 48 | 47 | 47 |
| Potato Flakes | 85 | 81 | 79 | 84 |
| Energy Bar 1 | 31 | 31 | 35 | 31 |
| Energy Bar 2 | 36 | 37 | 37 | 36 |
| Energy Bar 3 | 27 | 35 | 35 | 34 |
| Energy Bar 4 | 40 | 35 | 36 | 35 |
| Coca-Cola | 58 | 53 | 55 | 53 |
| Whole Milk | 40 | 41 | 40 | 41 |
| Clear Unsweetened Apple Juice | 44 | 50 | 46 | 48 |
| Glucose | 100 | 95 | 102 | 96 |
| Sucrose | 61 | 58 | 62 | 59 |
| Fructose | 19 | 21 | 20 | 25 |
| Lactose | 46 | 45 | 47 | 47 |
| Maltitol | 35 | 34 | 35 | 35 |
| Galactose | 20 | 20 | 20 | 20 |
| * Since the cereals used in Examples 2 and 3 were different, they were omitted from this data set. ** In vivo values were taken from Example 3 where they were derived as explained therein. | | | | |

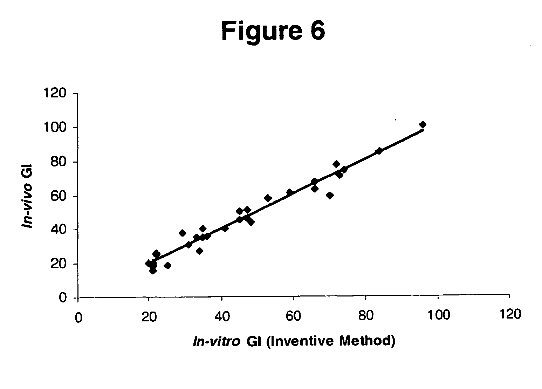

A scatter plot of this data obtained using the neural net curve fitting procedure is shown in Figure 6. A $R^2$ value of 0.96 is obtained using this method. Thus, again this method appears to have very high precision and high predictive value. The results of Examples 3 and 4, based on different number of samples but all run under similar conditions, generally agree.

As demonstrated in these Examples (especially in Examples 3 and 4), as the number of samples increases, the values of the various coefficients and other parameters used in the various curve fitting procedures are expected to be refined to achieve the best fit for the data set. Additional samples added to the data set are expected to increase the diversity of food products and better represent larger populations of food products.

**EP 2 171 472 B1**

**Claims**

1. An *in vitro* method for determining glycemic index for a test food product, said method comprising

   (1) determining protein content and fat content of the test food product;
   (2) providing the test food product in an essentially homogeneous and finely divided state to provide a test food sample;
   (3) digesting a sufficient amount of the test food sample in the essentially homogeneous and finely divided state to provide a standardized amount of available carbohydrate with a mixture of digestive enzymes for a fixed period of time to provide a digested sample;
   (4) treating the digested sample in its entirety immediately upon the fixed period of time to stop enzymatic reactions;
   (5) determining amounts of glucose and at least two sugars or sugar alcohols selected from the group consisting of fructose, galactose, lactose, sucrose, and maltitol in the digestive sample from step (4); and
   (6) calculating the glycemic index of the test food product from data obtained in steps (1) and (5) for the test food product using a predictive equation or a predictive method derived from multivariate analysis of calibration data for calibration samples wherein the calibration data is of same type and obtained in same manner as the data for the test food product determined in steps (1) and (5) and wherein the calibration samples have known in vivo glycemic index values.

2. The *in vitro* method of claim 1, wherein the amounts of all listed sugars and sugars alcohols in step (4) are determined and used to calculate the glycemic index in step (6).

3. The *in vitro* method of claim 1, wherein the test food product is obtained in the essentially homogeneous and finely divided state by grinding the test food product at a temperature below about -40°C.

4. The *in vitro* method of claim 2, wherein the test food product is obtained in the essentially homogeneous and finely divided state by grinding the test food product at a temperature of about -78°C or less.

5. The *in vitro* method of claim 1, wherein test food product is obtained in the essentially homogeneous and finely divided state by grinding the test food product at a temperature at or near liquid nitrogen temperatures.

6. The *in vitro* method of claim 2, wherein test food product is obtained in the essentially homogeneous and finely divided state by grinding the test food product at a temperature at or near liquid nitrogen temperatures.

7. The *in vitro* method of claim 1, wherein the amounts of glucose and the at least two sugars or sugar alcohols selected from the group consisting of fructose, galactose, lactose, sucrose, and maltitol in the digestive sample from step (4) are determined using high performance ion chromatography.

8. The *in vitro* method of claim 2, wherein the amounts of glucose and the sugars or sugar alcohols in the digestive sample from step (4) are determined using high performance ion chromatography.

9. The *in vitro* method of claim 1, wherein the test food product is obtained in the essentially homogeneous and finely divided state by grinding the test food product at a temperature below about -40°C and wherein the amounts of glucose and the at least two sugars or sugar alcohols selected from the group consisting of fructose, galactose, lactose, sucrose, and maltitol in the digestive sample from step (4) are determined using high performance ion chromatography.

10. The *in vitro* method of claim 1 wherein in step (2) the test food sample is provided in an essentially homogeneous and finely divided state by grinding the test food product at or near liquid nitrogen temperatures.

11. The *in vitro* method according to claim 10 wherein in step (5) amounts of glucose, fructose, galactose, lactose, sucrose, and maltitol in the digestive sample from step (4) are determined using high performance ion chromatography.

12. The *in vitro* method of claim 10 or 11, wherein the multivariate analysis uses a curve fitting procedure selected from the group consisting of multiple linear regression, partial least squares, and neural net.

**13.** The *in vitro* method of claim 10 or 11, wherein the curve fitting procedure is multiple linear regression.

**14.** The *in vitro* method of claim 10 or 11, wherein the curve fitting procedure is partial least squares.

**15.** The *in vitro* method of claim 10 or 11, wherein the curve fitting procedure is neural net.

**Patentansprüche**

**1.** *In vitro* Verfahren zur Ermittlung des glykämischen Index eines Nahrungsmitteltestproduktes, wobei das Verfahren umfasst:

(1) Ermitteln des Proteingehalts und Fettgehalts des Nahrungsmitteltestprodukts;
(2) Bereitstellen des Nahrungsmitteltestprodukts in einem hauptsächlich homogenen und fein verteilten Zustand, um eine Nahrungsmitteltestprobe bereitzustellen;
(3) Verdauen einer ausreichenden Menge der Nahrungsmitteltestprobe in dem hauptsächlich homogenen und fein verteilten Zustand zur Bereitstellung einer genormten Menge verfügbarer Kohlenhydrate mit einem Gemisch von Verdauungsenzymen für eine vorgegebene Zeitspanne, um eine verdaute Probe bereitzustellen;
(4) Behandeln der gesamten verdauten Probe unmittelbar nach der vorgegebenen Zeitspanne, um die enzymatischen Reaktionen zu beenden;
(5) Ermitteln der Mengenanteile der Glucose und von zumindest zwei Zuckern oder Zuckeralkoholen, die unter Fructose, Galactose, Lactose, Saccharose und Maltitol ausgewählt sind, in der Verdauungsprobe aus Schritt (4); und
(6) Berechnen des glykämischen Index des Nahrungsmitteltestprodukts aus den in den Schritten (1) und (5) für das Testprodukt erhaltenen Daten unter Verwendung einer Prädiktionsgleichung oder eines Prädiktionsverfahrens, das von der multivariaten Analyse von Kalibrierungsdaten für Kalibrierungsproben abgeleitet ist, wobei die Kalibrierungsdaten von der gleichen Art sind und in der gleichen Weise erhalten wurden wie die in den Schritten (1) und (5) für das Testprodukt bestimmten Daten und wobei für die Kalibrierungsproben die Werte für den glykämischen Index *in vivo* bekannt sind.

**2.** *In vitro* Verfahren nach Anspruch 1, worin die Mengenanteile aller aufgeführten Zucker und Zuckeralkohole in Schritt (4) ermittelt und dazu verwendet werden, den glykämischen Index in Schritt (6) zu berechnen.

**3.** *In vitro* Verfahren nach Anspruch 1, worin das Nahrungsmitteltestprodukt in dem hauptsächlich homogenen und fein verteilten Zustand erhalten wird, indem das Nahrungsmitteltestprodukt bei einer Temperatur unter -40 °C gemahlen wird.

**4.** *In vitro* Verfahren nach Anspruch 2, worin das Nahrungsmitteltestprodukt in dem hauptsächlich homogenen und fein verteilten Zustand erhalten wird, indem das Nahrungsmitteltestprodukt bei einer Temperatur von etwa -78 °C oder darunter gemahlen wird.

**5.** *In vitro* Verfahren nach Anspruch 1, worin das Nahrungsmitteltestprodukt in dem hauptsächlich homogenen und fein verteilten Zustand erhalten wird, indem das Nahrungsmitteltestprodukt bei einer Temperatur gemahlen wird, die der Temperatur von flüssigem Stickstoff entspricht oder in deren Nähe liegt.

**6.** *In vitro* Verfahren nach Anspruch 2, worin das Nahrungsmitteltestprodukt in dem hauptsächlich homogenen und fein verteilten Zustand erhalten wird, indem das Nahrungsmitteltestprodukt bei einer Temperatur gemahlen wird, die der Temperatur von flüssigem Stickstoff entspricht oder in deren Nähe liegt.

**7.** *In vitro* Verfahren nach Anspruch 1, worin die Mengenanteile der Glucose und der zumindest zwei Zucker oder Zuckeralkohole, die unter Fructose, Galactose, Lactose, Saccharose und Maltitol ausgewählt sind, in der Verdauungsprobe aus Schritt (4) unter Verwendung der Hochleistungsionenchromatographie ermittelt werden.

**8.** *In vitro* Verfahren nach Anspruch 2, worin die Mengenanteile der Glucose und der Zucker oder Zuckeralkohole in der Verdauungsprobe aus Schritt (4) unter Verwendung der Hochleistungsionenchromatographie ermittelt werden.

**9.** *In vitro* Verfahren nach Anspruch 1, worin das Nahrungsmitteltestprodukt in dem hauptsächlich homogenen und fein verteilten Zustand erhalten wird, indem das Nahrungsmitteltestprodukt bei einer Temperatur unter -40 °C gemahlen wird, und worin die Mengenanteile der Glucose und der zumindest zwei Zucker oder Zuckeralkohole, die unter Fructose, Galactose, Lactose, Saccharose und Maltitol ausgewählt sind, in der Verdauungsprobe aus Schritt (4) unter Verwendung der Hochleistungsionenchromatographie ermittelt werden.

10. *In vitro* Verfahren nach Anspruch 1, worin in Schritt (2) die Nahrungsmitteltestprobe in dem hauptsächlich homogenen und fein verteilten Zustand bereitgestellt wird, indem das Nahrungsmitteltestprodukt bei oder nahe der Temperatur von flüssigem Stickstoff zerkleinert wird.

11. *In vitro* Verfahren nach Anspruch 10, worin in Schritt (5) die Mengenanteile von Glucose, Fructose, Galactose, Lactose, Saccharose und Maltitol in der Verdauungsprobe aus Schritt (4) unter Verwendung der Hochleistungsionenchromatographie ermittelt werden.

12. *In vitro* Verfahren nach Anspruch 10 oder 11, worin die multivariate Analyse ein Kurvenanpassungsverfahren verwendet, das unter multipler linearer Regression, Partial Least Squares-Verfahren und neuronalem Netz ausgewählt ist.

13. *In vitro* Verfahren nach Anspruch 10 oder 11, worin es sich bei dem Kurvenanpassungsverfahren um multiple lineare Regression handelt.

14. *In vitro* Verfahren nach Anspruch 10 oder 11, worin es sich bei dem Kurvenanpassungsverfahren um das Partial Least Squares-Verfahren handelt.

15. *In vitro* Verfahren nach Anspruch 10 oder 11, worin es sich bei dem Kurvenanpassungsverfahren um ein neuronale Netz handelt.

**Revendications**

1. Procédé in vitro visant à déterminer un indice glycémique d'un produit alimentaire d'essai, ledit procédé comprenant les étapes qui consistent à

   (1) déterminer une teneur en protéines et en matières grasses du produit alimentaire d'essai ;
   (2) fournir le produit alimentaire d'essai dans un état sensiblement homogène et finement divisé afin de fournir un échantillon alimentaire d'essai ;
   (3) digérer une quantité suffisante de l'échantillon alimentaire d'essai dans l'état sensiblement homogène et finement divisé afin de fournir une quantité standardisée d'hydrates de carbone disponibles avec un mélange d'enzymes digestives pendant une période déterminée afin de donner un échantillon digéré ;
   (4) traiter l'échantillon digéré dans son intégralité immédiatement à la fin de la période déterminée afin d'arrêter les réactions enzymatiques ;
   (5) déterminer des quantités de glucose et d'au moins deux sucres ou d'alcools glucidiques sélectionnés du groupe composé de fructose, de galactose, de lactose, de saccharose, et de maltitol dans l'échantillon digestif à partir de l'étape (4) ; et
   (6) calculer l'indice glycémique du produit alimentaire d'essai d'après les données obtenues dans les étapes (1) et (5) pour le produit alimentaire d'essai en utilisant une équation de prédiction ou un procédé de prédiction dérivé d'une analyse multifactorielle des données d'étalonnage pour des échantillons d'étalonnage où les données d'étalonnage sont du même type et obtenues de la même manière que les données pour le produit alimentaire d'essai déterminées dans les étapes (1) et (5) et où les échantillons d'étalonnage ont des valeurs d'indice glycémique *in vivo*.

2. Procédé in vitro de la revendication 1, où les quantités de tous les sucres et les alcools glucidiques énumérés à l'étape (4) sont déterminées et utilisées pour calculer l'indice glycémique dans l'étape (6).

3. Procédé in vitro de la revendication 1, où le produit alimentaire d'essai est obtenu à l'état sensiblement homogène et finement divisé par broyage du produit alimentaire d'essai à une température inférieure à environ -40°C.

4. Procédé in vitro de la revendication 2, où le produit alimentaire d'essai est obtenu à l'état sensiblement homogène et finement divisé par broyage du produit alimentaire d'essai à une température inférieure ou égale à environ -78°C.

**5.** Procédé in vitro de la revendication 1, où un produit alimentaire d'essai est obtenu à l'état sensiblement homogène et finement divisé par broyage du produit alimentaire d'essai à une température proche ou égale à des températures de l'azote liquide.

**6.** Procédé in vitro de la revendication 2, où un produit alimentaire d'essai est obtenu à l'état sensiblement homogène et finement divisé par broyage du produit alimentaire d'essai à une température proche ou égale à des températures de l'azote liquide.

**7.** Procédé in vitro de la revendication 1, où les quantités de glucose et d'au moins deux sucres ou d'alcools glucidiques sélectionnés du groupe composé de fructose, de galactose, de lactose, de saccharose, et de maltitol dans l'échantillon digestif à partir de l'étape (4) sont déterminées par chromatographie ionique à haute performance.

**8.** Procédé in vitro de la revendication 2, où les quantités de glucose et les sucres ou alcools glucidiques dans l'échantillon digestif à partir de l'étape (4) sont déterminées par chromatographie ionique à haute performance.

**9.** Procédé in vitro de la revendication 1, où le produit alimentaire d'essai est obtenu à l'état sensiblement homogène et finement divisé par broyage du produit alimentaire d'essai à une température inférieure à environ -40°C et où les quantités de glucose et d'au moins deux sucres ou d'alcool glucidiques sélectionnées du groupe constitué de fructose, de galactose, de lactose, de saccharose, et de maltitol dans l'échantillon digestif à partir de l'étape (4) sont déterminées par chromatographie ionique à haute performance.

**10.** Procédé in vitro de la revendication 1, où dans l'étape (2) l'échantillon de produit alimentaire d'essai est fourni dans un état sensiblement homogène et finement divisé par broyage du produit alimentaire d'essai à une température proche ou égale à des températures de l'azote liquide.

**11.** Procédé in vitro selon la revendication 10, où dans l'étape (5) des quantités de glucose, de fructose, de galactose, de lactose, de saccharose, et de maltitol dans l'échantillon digestif de l'étape (4) sont déterminées par chromatographie ionique à haute performance.

**12.** Procédé in vitro de la revendication 10 ou 11, où l'analyse multifactorielle utilise une procédure d'ajustement des courbe sélectionnée du groupe constitué par une procédure par régression linéaire multiple, par moindres carrés partiels, et par réseau de neurones.

**13.** Procédé in vitro de la revendication 10 ou 11, où la procédure d'ajustement de courbe est une procédure de régression linéaire multiple.

**14.** Procédé in vitro de la revendication 10 ou 11, où la procédure d'ajustement de courbe est une procédure des moindres carrés partiels

**15.** Procédé in vitro de la revendication 10 ou 11, où la procédure d'ajustement de courbe est une procédure par réseau de neurones.

**Figure 1
(Prior Art)**

```
┌──────────────┐
│  Sample &    │
│  guar gum    │
└──────┬───────┘
       │
       ▼
┌──────────────┐
│  Pepsin in   │
│     HCL      │
└──────┬───────┘
       │
       ▼
┌──────────────┐
│ Sodium Acetate│
│  & Enzyme    │
│   Mixture    │
└──────┬───────┘
       │
       ▼
┌──────────────┐
│ Incubate at  │
│    37°C      │
└──────┬───────┘
       │
       ▼
┌──────────────┐      ┌──────────────┐      ┌──────────────┐
│  Sample at   │─────▶│  Ethanol;    │─────▶│   Measure    │  Aliquot 1
│   20 min.    │      │  centrifuge  │      │ glucose (G20)│
└──────┬───────┘      └──────────────┘      └──────────────┘
       │
       ▼
┌──────────────┐      ┌──────────────┐      ┌──────────────┐
│  Sample at   │─────▶│  Ethanol;    │─────▶│   Measure    │
│   120 min    │      │  centrifuge  │      │ glucose (G120)│  Aliquot 2
└──────┬───────┘      └──────────────┘      └──────────────┘
       │
       ▼
┌──────────────┐
│  Vortex Mix  │
└──────┬───────┘
       │
       ▼
┌──────────────┐                            ┌──────────────┐
│  30 min at   │                            │ Measure total│
│    100°C     │                            │ glucose (TG) │
└──────┬───────┘                            └──────▲───────┘
       │                                           │
       ▼                                           │
┌──────────────┐                            ┌──────────────┐
│ Cool to 0°C; │                            │ Cool, dilute │
│ add KOH; mix │                            │ & centrifuge │
│    30 min    │                            └──────▲───────┘
└──────┬───────┘                                   │
       │                                           │
       ▼                                           │
┌──────────────┐      ┌──────────────┐      ┌──────────────┐
│ Add to acetic│─────▶│  30 min at   │─────▶│  10 min at   │
│ acid; add    │      │    70°C      │      │    100°C     │
│amyloglucosidase│    └──────────────┘      └──────────────┘
└──────────────┘
```

## Figure 2

**Figure 3**

# Figure 4

A

*In vivo* GI vs *In-vitro* GI (Inventive Method)

B

*In vivo* GI vs RAG/% Available Carbohydrate

C

*In vivo* GI vs RAG (g glucose released/100g food)

# Figure 5

A

In-vivo GI vs In-vitro GI (Inventive Method)

B

In-vivo GI vs RAG/% Available Carbohydrate

C

In-vivo GI vs RAG (g glucose released/100g food)

# Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ENGLYST et al.** *Brit. J. Nutr.,* 1996, vol. 75, 327-337
- **ENGLYST et al.** *Am. J. Clin. Nutr.,* 1999, vol. 69, 448-454
- **GARSETTI et al.** *J. Am. Coll. Nutr.,* 2005, vol. 24, 441-447
- **BRAND-MILLER et al.** *Eur. J. Clin. Nutr.,* 2004, vol. 58, 700-701
- **FLINT ANNE et al.** The use of glycaemic index tables to predict glycaemic index of composite breakfast materials. *British Journal of Nutrition,* June 2004, vol. 91 (6), 979-989
- **TROUT DAVID et al.** Prediction of glycaemic index among high-sugar, low-starch foods. *International Journal of Food Sciences and Nutrition,* March 1999, vol. 50 (2), 135-144
- **ENGLYST CLAUS N et al.** Rapidly available glucose in foods; an in vitro measurement that reflects the glycemic response. *American Journal of Clinical Nutrition,* 01 March 1999, vol. 69 (3), 448-454
- *Am. J. Clin. Nutr.,* 2002, vol. 76, 5-56
- International table of glycemic index and glycemic load values. *Am. J. Clin. Nutr.,* vol. 76, 5-56
- **LIVESY.** *Nutr. Res. Rev.,* 2003, vol. 16 (2), 163-91
- **JENTJENS et al.** *Eur. J. App.l Physiol.,* 2003, vol. 88, 459-465
- International table of glycemic index and glycemic load values. *Am. J. Clin. Nutr.,* 2002, vol. 76, 5-56
- **JENTJENS et al.** *Eur. J. App. Physiol.,* 2003, vol. 88, 459-465